# EUROPEAN PATENT APPLICATION

(11) **EP 3 181 113 A1**
(43) Date of publication of application: **21.06.2017**
(21) Application number: 15199791.3
(22) Date of filing: 14.12.2015
(51) Int. Cl.: A61K 8/36, A61Q 5/10

(54) **HAIR COLORING COMPOSITION COMPRISING ONE OR MORE ACRYLIC COMPOUNDS**

(71) Applicant: The Procter & Gamble Company, Cincinnati, OH 45202 (US)
(72) Inventor: FLOHR, Andreas, 65824 Schwalbach am Taunus (DE); KRIPP, Thomas, 65824 Schwalbach am Taunus (DE)
(74) Representative: Sauvaître, Thibault Bruno

(57) **Abstract**

A hair coloring composition is provided and comprises, in a cosmetically acceptable carrier:
one or more acrylic compounds of Formula I, or a cosmetically acceptable salt thereof, or a mixture thereof: 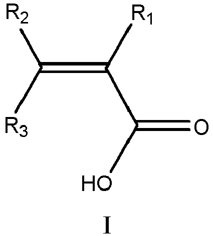 wherein
R₁
is selected from the group consisting of -H, -CH₃, -CF₃, -C₂H₅, -C₃H₈, -iPr, -C₄H₉, -sBu,-iBu, tBu-, and -CH₂-COOCH₃;
R₂
is selected from the group consisting of -H, -CH₃, -C₂H₅, -C₃H₈, -iPr, -C₄H₉, -sBu, -iBu, tBu-, -OCH₃, -COOH, and -CH₂COOH;
R₃
is selected from the group consisting of -H, and -CH₃.

## Description

### FIELD OF THE INVENTION

A hair coloring composition comprising in a cosmetically acceptable carrier, one or more acrylic compounds and oxidative dye precursors is provided for improving the vibrancy of a hair color. Also, a kit for coloring hair is provided and comprises the hair coloring composition and an oxidizing formulation which are separately packaged. A method of coloring hair is also provided.

### BACKGROUND OF THE INVENTION

Hair coloring or dyeing involves the application of one or more hair dyes onto hair which results in the coloration of hair fibers. The total head of hair color may be changed subtly or dramatically, the root growth colored to match the remaining head of hair, effects introduced such as glitter, hair swatch effects or other sectional effects, or the same color "freshened up" to combat fade and/or wash-out. In modem times, the consumer has a wide variety of options for coloring the hair (whether in the salon or at home) from direct dyes that wash out relatively quickly, hair make-up for applying glitter and/or hair swatch effects, to conventional (semi-) permanent dyeing technology.

Hair dyes are generally formulated to give hair long-lasting hued colors, but several factors from mechanical to environmental can accelerate color changes and fading. When the hair color is applied to the hair, the color molecules deposit throughout the cortex and cuticle regions of the fiber. The color molecules, however, do not remain permanently affixed inside the hair, rather as time passes, the color molecules diffuse out towards the surface of the fiber. As the color molecules diffuse towards the surface of the fiber, they come into more direct contact with water during washing. Therefore, the primary cause of color fading is due to the color diffusion and hair's contact with water. Washing both increases the rate of diffusion of the color molecules, as well as eventually washing the color molecules from the hair.

A method and a composition has been provided for preventing color loss in dyed hair through the application of a so-called color lock composition which provides enhanced efficacy and performance for preventing color loss from hair fibers, see for instance U.S. Patent 7,981,167 B2.

However, the method typically requires to carry out separately the step of dyeing hair from the step of applying the color lock composition comprising at least one ethylenic monomer such as sodium sulfopropyl acrylate.

There is still a need to provide the consumer not only with a composition that can prevent color loss but also with a composition that can improve the vibrancy (i.e. intensity, or relative deep color) of the color on the hair fibers.

There is also a need to develop a one-step method wherein the application of the composition improving the vibrancy of the color is simultaneously carried out with the application of hair color to the hair fibers.

### SUMMARY OF THE INVENTION

The present invention is related to a hair coloring composition which comprises, in a cosmetically acceptable carrier: one or more acrylic compounds of Formula I, or a cosmetically acceptable salt thereof, or a mixture thereof: 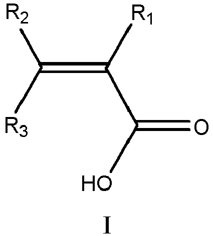 wherein
R₁ is selected from the group consisting of -H, -CH₃, -CF₃, -C₂H₅, -C₃H₈, -iPr, -C₄H₉, -sBu, - iBu, tBu-, and -CH₂-COOCH₃;
R₂ is selected from the group consisting of -H, -CH₃, -C₂H₅, -C₃H₈, -iPr, -C₄H₉, -sBu, -iBu, tBu-, -OCH₃, -COOH, and -CH₂COOH;
R₃ is selected from the group consisting of -H, and -CH₃;
The one or more acrylic compounds of Formula I have a molecular weight of less than 500 g/mol, preferably less than 300 g/mol, more preferably less than 250 g/mol or even more preferably less than 100 g/mol. The one or more acrylic compounds of Formula I have a molecular weight of from 70 g/mol to 500 g/mol, or preferably from 70 g/mol to 300 g/mol, or more preferably from 70 g/mol to 250 g/mol or even more preferably from 70 g/mol to 100 g/mol. The hair coloring composition comprises oxidative dye precursors comprising one or more couplers and one or more primary intermediates. The hair coloring composition has a pH of from 8 to 12.

The hair coloring composition may comprise from 0.5% to 50%, preferably from 0.5% to 15% of the one or more acrylic compounds of Formula I, or a cosmetically acceptable salt thereof, or a mixture thereof by total weight of the hair coloring composition

The hair coloring composition may comprise a polymerisation inhibitor. The polymerisation inhibitor may be selected from the group consisting of 4-methoxyphenol, nitrobenzene, diphenyl picryl hydrazyl (DPPH) and mixtures thereof.

Alternatively, the polymerisation inhibitor may be selected from the group consisting of 2-tert-butyl-4-hydroxy-anisole, 3-tert-butyl-4-hydroxy-anisole, and mixtures thereof.

A use of one or more acrylic compounds of Formula I or a cosmetically acceptable salt thereof, or a mixture thereof: 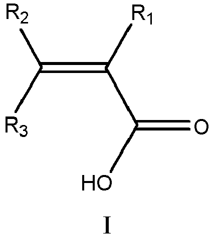 wherein
R₁ is selected from the group consisting of -H, -CH₃, -CF₃, -C₂H₅, -C₃H₈, -iPr, -C₄H₉, -sBu, - iBu, tBu-, and -CH₂-COOCH₃;
R₂ is selected from the group consisting of -H, -CH₃, -C₂H₅, -C₃H₈, -iPr, -C₄H₉, -sBu, -iBu, tBu-, -OCH₃, -COOH, and -CH₂COOH;
R₃ is selected from the group consisting of -H, and -CH₃;
in an hair coloring composition, wherein the one or more acrylic compounds of Formula I have a molecular weight of less than 500 g/mol, for improving the vibrancy of a hair color.

A kit for coloring hair is provided and comprises:
(a) a hair coloring composition according to the present invention;
(b) an oxidizing formulation comprising one or more oxidizing agents; and
wherein the hair coloring composition (a) and the oxidizing formulation (b) are separately packaged.

A method of coloring hair is provided and comprises:
(a) mixing a hair coloring composition according to the present invention with an oxidizing formulation comprising one or more oxidizing agents to form a mixture; and
(b) applying the mixture onto hair.

### BRIEF DESCRIPTION OF THE DRAWINGS

While the specification concludes with claims particularly pointing out and distinctly claiming the present invention, it is believed that the same will be better understood from the following description read in conjunction with the accompanying drawings in which:
Fig. 1 is a photography showing pairs of two hair swatches treated by the respective compositions A-F;
Fig. 2 is the L*a*b* color space obtained by measuring and averaging the L*a*b* values of two hair swatches that have been treated with each respective composition A-F as shown in Fig. 1.

### DETAILED DESCRIPTION OF THE INVENTION

### General and definitions

In this document, including in all embodiments of all aspects of the present invention, the following definitions apply unless specifically stated otherwise.

All percentages are by total weight (w/w) of the hair coloring composition, unless otherwise specified. All ratios are weight ratios. "% wt." means percentage by weight. References to 'parts' e.g. a mixture of 1 part X and 3 parts Y, is a ratio by weight. When more than one composition are used during a treatment, the total weight to be considered is the total weight of all the compositions applied on the hair simultaneously (i.e. the weight found "on head"), typically resulting from mixing an oxidative composition (also called developer and/or oxidizing composition/component) with a dye composition (also called tint, and/or dye composition/component), unless otherwise specified. All ratios or percentages are weight ratios or weight percentages unless specifically stated otherwise.

"QS" or "QSP" means sufficient quantity for 100% or for 100g. +/- indicates the standard deviation. All ranges are inclusive and combinable. The number of significant digits conveys neither a limitation on the indicated amounts nor on the accuracy of the measurements. All numerical amounts are understood to be modified by the word "about".

All measurements are understood to be made at 20°C and at ambient conditions, where "ambient conditions" means at 1 atmosphere (atm) of pressure and at 65% relative humidity, unless otherwise stated. "Relative humidity" refers to the ratio (stated as a percent) of the moisture content of air compared to the saturated moisture level at the same temperature and pressure. Relative humidity can be measured with a hygrometer, in particular with a probe hygrometer from VWR^{®} International.

Herein "min" means "minute" or "minutes". Herein "mol" means mole. Herein "g" following a number means "gram" or "grams". "Ex." means "example". All amounts as they pertain to listed ingredients are based on the active level ('solids') and do not include carriers or by-products that may be included in commercially available materials.

Herein, "comprising" means that other steps and other ingredients can be in addition. "Comprising" encompasses the terms "consisting of" and "consisting essentially of'. The compositions, formulations, methods, uses, kits, and processes of the present invention can comprise, consist of, and consist essentially of the elements and limitations of the invention described herein, as well as any of the additional or optional ingredients, components, steps, or limitations described herein. Embodiments and aspects described herein may comprise or be combinable with elements, features or components of other embodiments and/or aspects despite not being expressly exemplified in combination, unless an incompatibility is stated.

Where amount ranges are given, these are to be understood as being the total amount of said ingredient in the composition, or where more than one species fall within the scope of the ingredient definition, the total amount of all ingredients fitting that definition, in the composition.

For example, if the composition comprises from 1% to 5% fatty alcohol, then a composition comprising 2% stearyl alcohol and 1% cetyl alcohol and no other fatty alcohol, would fall within this scope.

The amount of each particular ingredient (e.g. a primary intermediate, a coupler, an oxidizing agent, etc) or mixtures thereof described hereinafter can account for up to 100% (or 100%) of the total amount of the ingredient(s) in the hair coloring composition.

The term "substantially free of" as used herein means less than 1%, less than 0.8%, less than 0.5%, less than 0.3%, or less than an immaterial amount of by total weight of the composition or formulation.

The term "hair" as used herein means mammalian hair including scalp hair, facial hair and body hair, more preferably hair on the human head and scalp. Hair comprises hair fibers. "Hair shaft" means an individual hair swatch and may be used interchangeably with the term "hair." "Internal region of the hair shaft," as used herein, means any non-surface portion of the hair shaft, including the inner portion of the cuticle, underneath the cuticle and the cortex. "Non-surface portion" may be understood to mean that portion of the hair that is not in direct contact with the outside environment. As used herein the term "hair" to be treated may be "living" i.e. on a living body or may be "non-living" i.e. in a wig, hairpiece or other aggregation of non-living keratinous fibers. Mammalian, preferably human hair is preferred. However wool, fur and other keratin containing fibers are suitable substrates for the hair coloring compositions according to the present invention.

By "hair coloring composition", it is meant a composition suitable for changing the color of hair. The hair coloring composition is referred hereinafter as "the composition", unless otherwise specified. The hair coloring composition can comprise oxidative dye precursors, direct dyes. The term "hair coloring composition" as used herein covers hair bleaching and hair oxidative dyeing products.

The term "cosmetically acceptable" as used herein means that the compositions, formulations or components described are suitable for use in contact with human keratinous tissue without undue toxicity, incompatibility, instability, allergic response, and the like. All compositions and formulations described herein which have the purpose of being directly applied to keratinous tissue are limited to those being cosmetically acceptable.

The term "derivatives" as used herein includes but is not limited to: ester, amide, carboxyl, amino, ether, acetyl, acid, salt and/or alcohol or hydroxy derivatives of a given compound.

The term "monomer" as used herein means a discrete, non-polymerised chemical moiety capable of undergoing polymerisation in the presence of an initiator. "Ethylenic monomer", as used herein, means a monomer that contains an olefinic carbon-carbon double bond (C=C) and is capable of undergoing polymerisation, preferably radical polymerisation, in the presence of an initiator.

The term "polymer" as used herein means a chemical formed from the polymerisation of two or more monomers. The term "polymer" as used herein shall include all materials made by the polymerisation of monomers as well as natural polymers. Polymers made from only one type of monomer are called homopolymers. A polymer comprises at least two monomers. Polymers made from two or more different types of monomers are called copolymers. The distribution of the different monomers can be calculated statistically or block-wise. Except if stated otherwise, the term "polymer" used herein includes any type of polymer including homopolymers and copolymers.

The term "molecular weight of a polymer" or "M.Wt. of a polymer" as used herein refers to the weight average molecular weight unless otherwise stated. The weight average molecular weight may be measured by gel permeation chromatography.

The term "cosmetically acceptable salt" as used herein refers to conventional base-addition salts that retain the properties of the one or more acrylic compounds of the present invention and are formed from suitable organic or inorganic bases. Sample base-addition salts include those derived from sodium, potassium, ammonium, calcium, magnesium, iron, zinc, zirconium and aluminium hydroxide. Chemical modification of a compound bearing a carboxylic acid function into the corresponding carboxylate salt is a technique well known in the art.

The term "viscosity" as used herein is measured at 25°C using a HAAKE Rotation Viscometer VT 550 with cooling/heating vessel and sensor systems according to DIN 53019 at a shear rate of 12.9 s⁻¹.

The term "water-soluble" as used herein refers to any material that is sufficiently soluble in water to form a clear solution to the naked eye at a concentration of 0.1% by weight of the material in water at 25°C. The term "water-insoluble" refers to any material that is not "water-soluble".

The term "separately packaged" as used herein means any form of packaging that prevents a first composition or formulation from coming into physical contact, or admixing, with a second composition or formulation. "Separately packaged" may mean that the individual first and second compositions are packaged in separate containers, or alternatively in a single container partitioned such that the first and second compositions are not in physical contact.

The term "kit" as used herein means a packaging unit comprising a plurality of components i.e. a kit of parts. An example of a kit is, for example, a first composition and a separately packaged second composition. Another kit may comprise application instructions comprising a method and a composition/formulation.

### Hair coloring composition

The present invention is related to a hair coloring composition as stated hereinbefore. The hair coloring composition comprises in a cosmetically acceptable carrier, one or more of acrylic compounds as described more in detail below.

### Acrylic compound

It has been found that a color lock composition comprising an ethylenic monomer, e.g. sulfopropyl acrylic acid forms a polymer inside the hair fiber which slows the loss of the color molecule(s) resulting from: i) filling up the channels in the hair cuticle, thus blocking the path for diffusion of the color molecules to the hair surface, and ii) by stably affixing to the color molecule(s) thus slowing the rate of diffusion or the color molecules to the fiber surface.

However, it has been found when the color lock composition is simultaneously applied with oxidative dye precursors, which are pre-mixed with an adequate oxidizing formulation, on the hair fibers, the resulting dyeing led to a relatively lighter shade. It is believed that the ethylenic monomer might interact with the oxidative dye precursors. Hence, a two-step method consisting of the application of the color lock composition in a first step followed by the application of the oxidative dye precursors with an adequate oxidizing agent in a second step has been implemented.

It has been surprisingly found that it is possible to improve the vibrancy of the color by replacing the ethylenic monomer by one or more acrylic compounds of Formula I: 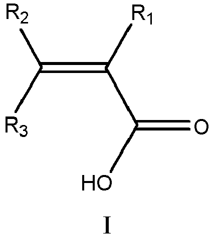 wherein
R₁ is selected from the group consisting of -H, -CH₃, -CF₃, -C₂H₅, -C₃H₈, -iPr, -C₄H₉, -sBu, - iBu, tBu-, and -CH₂-COOCH₃;
R₂ is selected from the group consisting of -H, -CH₃, -C₂H₅, -C₃H₈, -iPr, -C₄H₉, -sBu, -iBu, tBu-, -OCH₃, -COOH, and -CH₂COOH;
R₃ is selected from the group consisting of -H, and -CH₃.

While not wishing to be bound by theory, it is believed that at a relatively high pH, the one or more acrylic compounds of Formula I do not polymerize. Indeed, the deprotonated carboxylic acid is not prone to radical polymerization.

Hair is typically damaged due to oxidative color treatment. As a result, hair fibers comprise a plurality of pores. As the one or more acrylic compounds of Formula I are not prone to polymerization at a relatively high pH, as it has a relatively small size and a good water solubility, the one or more acrylic compounds can penetrate and fill the pores of the damaged hair fibers.

The presence of the one or more acrylic compounds of Formula I can help to promote ring dyeing to occur, i.e. a relatively intense hair color is obtained with a very moderate color shift. Unlike with the color lock composition requiring a two-step method, now the one or more acrylic compounds of Formula I can be simultaneously applied with oxidative dye precursors on the hair fibers. Hence, the hair coloring composition comprising the one or more acrylic compounds of Formula I can provide a relative enhanced color vibrancy, i.e. a relatively higher intensity of the color obtaining from the same hair coloring composition but without any acrylic compounds of Formula I.

Also, as a relatively intense hair color is obtained with a very moderate color shift, it is possible to reduce the relative amount of oxidative dye precursors in the hair coloring composition. Indeed, the inventors have found that with the hair coloring composition of the present invention, it is possible to obtain a similar color than the color obtained when using a hair coloring composition comprising only the same oxidative dye precursors at a relative higher concentration (without using any acrylic compound) which are pre-mixed with an adequate oxidizing formulation. Hence, relative lower total amount of one or more couplers and one or more primary intermediates is needed to obtain the similar color (i.e. with no color shift), which can appears to be a more effective and cost saving hair coloring composition. In that case, hair is also advantageously less exposed to oxidative dye precursors, which can help to preserve hair from oxidative damage.

While not wishing to be bound by theory, it is believed that the pores of the hair fibers are filled with the one or more acrylic compounds while the oxidative dye precursors react with each other upon oxidation with the one or more oxidizing agents of the adequate oxidizing formulation. The same color but with an improved vibrancy, i.e. with a relative higher intensity, as the one obtained using a hair coloring composition comprising only the oxidative dye precursors without any acrylic compound is obtained. With the hair coloring composition of the present invention, the dye is formed in the perimeter of the hair shaft, which gives an impression of deeper color to the consumer, namely an improvement of the vibrancy of the color.

The different aspects of the present invention will now be discussed in more details.

The hair coloring composition comprises in a cosmetically acceptable carrier, one or more acrylic compounds selected from the group consisting of one or more acrylic compounds of Formula I, a cosmetically acceptable salt thereof and mixtures thereof: 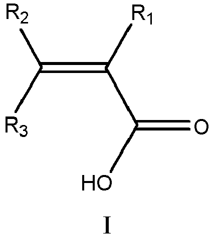 wherein
- R₁: is -H, -CH₃, -CF₃, -C₂H₅, -C₃H₈, -iPr, -C₄H₉, -sBu, -iBu, tBu-, -CH₂-COOCH₃;
- R₂: is -H, -CH₃, -C₂H₅, -C₃H₈, -iPr, -C₄H₉, -sBu, -iBu, tBu-, -OCH₃, -COOH, CH₂COOH; and
- R₃: is -H, -CH₃,
-iPr being isopropyl group, -sBu being sec-butyl group, -iBu being isobutyl group, and tBu-being tertio-butyl group.

The hair coloring composition may comprise in a cosmetically acceptable carrier, from 0.5% to 50% or from 0.5% to 15%, or from 1% to 15%, or from 1% to 10%, or from 1% to 5% of the one or more acrylic compounds of Formula I, or a cosmetically acceptable salt thereof or a mixture thereof by total weight of the hair coloring composition.

The one or more acrylic compounds of Formula I have a molecular weight of less than 500 g/mol, preferably less than 300 g/mol, more preferably less than 250 g/mol, or even more preferably less than 100 g/mol. The one or more acrylic compounds of Formula I may be water-soluble.

The hair coloring composition has a pH from 8 to 12, or from 9 to 10. As set out above, the one or more acrylic compounds of Formula I are not prone to radical polymerisation at a relatively high pH. It may be nevertheless advantageous to ensure that the one or more acrylic compounds remain exclusively as such i.e. as an unsaturated acrylic compound, by employing a polymerisation inhibitor in the hair coloring composition.

The hair coloring composition may comprise a polymerisation inhibitor. The hair coloring composition may comprise a polymerisation inhibitor in an amount of from 1 milligram to 1000 milligram per kilogram of the one or more acrylic compounds having the general Formula I.

The polymerisation inhibitor may be selected from the group consisting of 4-methoxyphenol, nitrobenzene, 2,2-diphenyl-l-picrylhydrazyl (DPPH) and mixtures thereof. For instance, Irganox^{®} from BASF may be used. Irganox^{®} 1330 is a Tris-BHT Mesitylene compound. Ethanox from SI Group is a non-coloring, odourless antioxidant. For example, ETHANOX 330 or 330G may also be used.

Alternatively, the polymerisation inhibitor may be selected from the group consisting of 2-tert-butyl-4-hydroxy-anisole, 3-tert-butyl-4-hydroxy-anisole, and mixtures thereof. Regarding the hydroxyl anisole compound as a polymerisation inhibitor, it is believed that upon any formation of a carbon radical from the double bond of the one or more acrylic compounds of Formula I, the radical of the one or more acrylic compounds can be trapped by the proton provided from the hydroxyl group of the anisole compound. Then, the resulting acrylic compound is unable to polymerise since a radical is no longer present.

The hair coloring composition may be substantially free of any initiator of free-radical polymerisation. Indeed, the hair coloring composition being substantially free of any initiator of free-radical polymerisation has the advantage that the risk of the one or more acrylic compounds polymerising with itself/themselves in the hair coloring composition is reduced.

The hair coloring composition may be substantially free of polyacrylic polymer derivative(s) obtained from the one or more acrylic compounds of the present invention. This may be with the exception of any polyacrylic polymer derivative(s) employed in any particles, such as capsules that could be used for encapsulating the hair coloring composition of the present invention.

The one or more acrylic compounds of the hair coloring composition may preferably conform to the Formula II, or a cosmetically acceptable salt, or a mixture thereof: 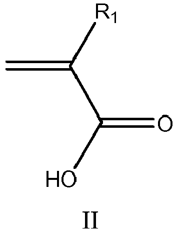 wherein
R₁ is selected from the group consisting of -H, -CH₃, -CF₃, -C₂H₅, -C₃H₈, -iPr, -C₄H₉, -sBu, - iBu, tBu-, and -CH₂-COOCH₃;
- R₂: is H;
- R₃: is H.

Alternatively, the one or more acrylic compounds of the hair coloring composition may more preferably conform to the general Formula III, or a cosmetically acceptable salt thereof, or a mixture thereof: 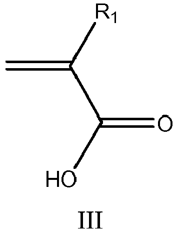 wherein
- R₁: is -H, -CH₃;
- R₂: is H;
- R₃: is H.

The one or more acrylic compounds may be added to the hair coloring composition as 2-propenic acid (acrylic acid) or 2-methylpropenic acid (methacrylic acid), or in a cosmetically acceptable salt thereof of 2-propenic acid (acrylic acid) or 2-methylpropenic acid (methacrylic acid).

For any acrylic compound of the present invention, the one or more acrylic compounds may be in a cosmetically acceptable salt. The cosmetically acceptable salt may be selected from the group consisting of sodium salt, potassium salt, ammonium salt, calcium salt, magnesium salt, iron salt, zinc salt, zirconium salt, aluminium salt and mixtures thereof. The base-addition salts can retain the properties of the one or more acrylic compounds of the present invention and are formed from suitable organic or inorganic bases. Sample base-addition salts include those derived from sodium, potassium, ammonium, calcium, magnesium, iron, zinc, zirconium and aluminium hydroxide. A cosmetically acceptable salt of the one or more acrylic compounds of the present invention can help increasing its water-solubility.

The one or more acrylic compounds of Formula I, preferably of Formula II, more preferably of Formula III can be used in a hair coloring composition to enhance the vibrancy of the hair color at a very moderate color shift compared to the same hair coloring composition comprising only oxidative dye precursors.

As shown in the Examples below, for the same color (i.e. no color shift or at the most a very moderate color shift), when the hair coloring composition comprises one or more acrylic compounds of Formula I, preferably of Formula II, more preferably of Formula III, it is possible to reduce the relative amount of oxidative dye precursors in the hair coloring composition than the amount of oxidative dye precursors used in the hair coloring composition comprising only oxidative dye precursors. Using lower amount of oxidative dye precursors in a hair coloring composition appears to be a cost saving hair coloring composition. Also, with a relatively lower amount of oxidative dye precursors, the hair fibers and the head skin of the consumers can be advantageously less exposed to the oxidative dye precursors of the hair coloring composition, which can help to preserve hair from oxidative damage.

### pH

The hair coloring composition has a pH from 8 to 12. The pH of the hair coloring composition may be from 8.0 to 9.0, or from 9.1 to 10.0, or from 10.1 to 11.0, or from 11.1 to 12.0. Having a basic pH can help to prevent the polymerization of the one or more acrylic compounds of the hair coloring composition.

The hair coloring composition may comprise a pH modifier and/or buffering agent in an amount that is sufficiently effective to adjust the pH of the hair coloring composition to fall within a range prescribed above. Suitable pH modifiers and/or buffering agents for use herein may include, but are not limited to ammonia, alkanolamines such as monoethanolamine, diethanolamine, triethanolamine, monopropanolamine, dipropanolamine, tripropanolamine, 2-amino-2-methyl-1-propanol, and 2-amino-2-hydroxymethyl-1,3,-propandiol and guanidium salts, alkali metal or ammonium hydroxides and carbonates.

Suitable pH modifiers and/or buffering agents may preferably include sodium hydroxide, sodium silicate, sodium meta silicate and ammonium carbonate, and acidulents such as inorganic and inorganic acids, e.g., phosphoric acid, acetic acid, ascorbic acid, citric acid or tartaric acid, hydrochloric acid, and mixtures thereof. The pH of the hair coloring composition may be adjusted with ammonia or monoethanol amine.

### Alkalising agent

The hair coloring composition may comprise one or more alkalising agents. Typically, the hair coloring composition may comprise a total amount from 0.1% to 10%, or from 0.5% to 6%, or from 1% to 4%, of the one or more alkalizing agents by total weight of the hair coloring composition.

By "alkalising agent", it is meant one or more compounds suitable for increasing the pH to alkaline level. Generally, the most commonly used alkalising agent in the art is ammonia. Non-ammonia alkalising agents are also known and advantageous in view of reduced olfactory stimulation, e.g. alkanolamines.

The hair coloring composition may comprise one or more non-ammonia alkalizing agents selected from the group consisting of: monoethanolamine (MEA), sodium silicate, sodium meta silicate, diethanolamine, triethanolamine, monopropanolamine, dipropanolamine, tripropanolamine, 2-amino-2-methyl-1,3-propanediol, 2-amino-2-methyl-1-propanol (a.k.a. aminomethylpropanol, AMP), 2-amino-2-hydroxymethyl-1,3-propanediol, and mixtures thereof.

Monoethanolamine (MEA) or aminomethylpropanol (AMP) are commonly used in ammonia-free hair dye products and may be preferred as the alkalising agent alone or in combination with each other or other alkalizing agents. Monoethanolamine may in particular be preferred to be used alone or in combination with other non-ammonia alkalising agent. The hair coloring composition may comprise ammonia in addition to the one or more non-ammonia alkalising agents, for example less than 0.5% of ammonia by total weight of the hair coloring composition. The one or more alkalising agents of the hair coloring composition may be preferably monoethanolamine (MEA).

The hair coloring composition may comprise an alkalising agent which is monoethanolamine (MEA) and a primary intermediate which is 2-methoxymethyl-1,4-benzenediamine.

### Oxidative dye precursors

The hair coloring composition comprises oxidative dyes precursors comprising one or more couplers (also known as secondary intermediate) and one or more primary intermediates (also known as developer). Various couplers may be used with primary intermediates in order to obtain different shades.

The oxidative dye precursors suitable for use herein, in so far as they are bases, may be used as free bases or in the form of any cosmetically acceptable salts obtained with the corresponding organic or inorganic acids, such as hydrochloric, hydrobromic, citric, acetic, lactic, succinic, tartaric, or sulfuric acids, or, in so far as they have aromatic hydroxyl groups, in the form of any cosmetically acceptable salts obtained with the corresponding bases, such as alkali phenolates.

Oxidative dye precursors are known in the art, and include aromatic diamines, aminophenols, aromatic diols and their derivatives (a representative but not exhaustive list of oxidation dye precursors can be found in Sagarin, "Cosmetic Science and Technology, Interscience, Special Edn. Vol. 2 pages 308 to 310). Suitable oxidative dye precursors are also disclosed in the Canadian Patent Application No. CA2576189A1 - in particular, from Table 1 dye combinations No. 1 to 2394, which span pages 49 to 238, are incorporated herein by reference. It is to be understood that the one or more primary intermediates and the one or more couplers (collectively known as oxidative dye precursors) detailed below are only by way of example and are not intended to limit the hair coloring compositions and other aspects herein described. The one or more primary intermediates and the one or more couplers may be used in the form of any cosmetically acceptable salts, for example sulfate salts.

The one or more primary intermediates of the hair coloring composition may be selected from the group consisting of toluene-2,5-diamine, p-phenylenediamine, N-phenyl-p-phenylenediamine, N,N-bis(2-hydroxyethyl)-p-phenylenediamine, 2-hydroxyethyl-p-phenylenediamine, hydroxypropyl-bis-(N-hydroxyethyl-p-phenylenediamine), 2-methoxymethyl-p-phenylenediamine, 2-(1,2-dihydroxyethyl)-p-phenylenediamine, 2,2'-(2-(4-aminophenylamino)ethylazanediyl)diethanol, 2-(2,5-diamino-4-methoxyphenyl)propane-1,3-diol, 2-(7-amino-2H-benzo[b][1,4]oxazin-4(3H)-yl)ethanol, 2-chloro-p-phenylenediamine, p-aminophenol, p-(methylamino)phenol, 4-amino-m-cresol, 6-amino-m-cresol, 5-ethyl-o-aminophenol, 2-methoxy-p-phenylenediamine, 2,2'-methylenebis-4-aminophenol, 2,4,5,6-tetraminopyrimidine, 2,5,6-triamino-4-pyrimidinol, 1-hydroxyethyl-4,5-diaminopyrazole sulfate, 4,5-diamino-1-methylpyrazole, 4,5-diamino-1-ethylpyrazole, 4,5-diamino-1-isopropylpyrazole, 4,5-diamino-1-butylpyrazole, 4,5-diamino-1-pentylpyrazole, 4,5-diamino-1-benzylpyrazole, 2,3-diamino-6,7-dihydropyrazolo[1,2-a]pyrazol-1(5H)-one dimethosulfonate, 4,5-diamino-1-hexylpyrazole, 4,5-diamino-1-heptylpyrazole, methoxymethyl-1,4-diaminobenzene, N,N-bis(2-hydroxyethyl)-N-(4-aminophenyl)-1,2-diaminothane, 2-[(3-aminopyrazolo[1,5-a]pyridin-2-yl)oxy]ethanol hydrochloride, salts thereof and mixtures thereof.

The one or more primary intermediate of the hair coloring composition may be particularly 1,4-diamino-2-(methoxymethyl)-benzene. 1,4-diamino-2-(methoxymethyl)-benzene has the advantage of an improved sensitisation profile (i.e. reduced risks of scalp skin reaction).

The one or more primary intermediate may be 4,5-diamino-1-hexylpyrazole. 4,5-diamino-1-hexylpyrazole may be used as a sulfate salt.

The one or more primary intermediate may be selected from the group consisting of 4,5-diamino-1-butylpyrazole, 4,5-diamino-1-pentylpyrazole, 4,5-diamino-1-benzylpyrazole, 2,3-diamino-6,7-dihydropyrazolo[1,2-a]pyrazol-1(5H)-one dimethosulfonate, 4,5-diamino-1-hexylpyrazole, 4,5-diamino-1-heptylpyrazole, methoxymethyl-1,4-diaminobenzene, and mixtures thereof; and the cosmetically acceptable salts thereof such as chlorides, sulfates and hemi-sulfates in particular.

The one or more couplers may be a compound comprising at least one phenyl ring substituted with at least one hydroxyl group. The one or more couplers may be selected from the group consisting of resorcinol, 4-chlororesorcinol, 2-chlororesorcinol, 2-methylresorcinol, 4,6-dichlorobenzene-1,3-diol, 2,4-dimethylbenzene-1,3-diol, m-aminophenol, 4-amino-2-hydroxytoluene, 2-methyl-5-hydroxyethylaminophenol, 3-amino-2,6-dimethylphenol, 3-amino-2,4-dichlorophenol, 5-amino-6-chloro-o-cresol, 5-amino-4-chloro-o-cresol, 6-hydroxybenzomorpholine, 2-amino-5-ethylphenol, 2-amino-5-phenylphenol, 2-amino-5-methylphenol, 2-amino-6-methylphenol, 2-amino-5-ethoxyphenol, 5-methyl-2-(methylamino)phenol, 2,4-diaminophenoxyethanol, 2-amino-4-hydroxyethylaminoanisole, 1,3-bis-(2,4-diaminophenoxy)-propane, 2,2'-(2-methyl-1,3-phenylene)bis(azanediyl)diethanol, benzene-1,3-diamine, 2,2'-(4,6-diamino-1,3-phenylene)bis(oxy)diethanol, 3-(pyrrolidin-1-yl)aniline, 1-(3-(dimethylamino)phenyl)urea, 1-(3-aminophenyl)urea, 1-naphthol, 2-methyl-1-naphthol, 1,5-naphthalenediol, 2,7-naphthalenediol, 1-acetoxy-2-methylnaphthalene, 4-chloro-2-methylnaphthalen-1-ol, 4-methoxy-2-methylnaphthalen-1-ol, 2,6-dihydroxy-3,4-dimethylpyridine, 2,6-dimethoxy-3,5-pyridinediamine, 3-amino-2-methylamino-6-methoxypyridine, 2-amino-3-hydroxypyridine, 2,6-diaminopyridine, pyridine-2,6-diol, 5,6-dihydroxyindole, 6-hydroxyindole, 5,6-dihydroxyindoline, 3-methyl-1-phenyl-1H-pyrazol-5(4H)-one, 1,2,4-trihydroxybenzene, 2-(benzo[d][1,3]dioxol-5-ylamino)ethanol (also known as hydroxyethyl-3,4-methylenedioxyaniline), and mixtures thereof.

The oxidative dye precursors may be particularly selected from the group consisting of 1-naphthol, 2,4-diaminophenoxyethanol, toluene-2,5-diamine sulfate, resorcinol, 4-amino-m-cresol, 2-amino-6-chloro-4-nitrophenol, 2-amino-4-hydroxyethylaminoanisole sulfate, hydroxyethyl-3,4-methylenedioxyaniline HCl, 1-hydroxyethyl 4,5-diamino pyrazole sulfate, 4-amino-2-hydroxytoluene, 2-methylresorcinol, m-aminophenol, 2-methyl-5-hydroxyethylaminophenol, and mixtures thereof.

The oxidative dye precursors may comprise preferably 5-amino-4-chloro-o-cresol and 1,4-diamino-2-(methoxymethyl)-benzene. The oxidative dye precursors may comprise more preferably 2,6-diaminopyridine and 1,4-diamino-2-(methoxymethyl)-benzene. The oxidative dye precursors may comprise even more preferably 2,6-dihydroxyethylaminotoluene and 2-methoxymethyl-1,4-diaminobenzene. The oxidative dye precursors may comprise even more preferably 2-methoxymethyl-1,4-diaminobenzene and *p*-phenylenediamine and/or toluene-2,5-diamine.

Typically, the hair coloring composition may comprise a total amount of oxidative dye precursors, namely the one or more couplers and the one or more primary intermediates, up to 12%, or from 0.001% to 12%, or from 0.01% to 10%, or from 0.3% to 8%, or from 0.05% to 9%, or from 0.5% to 6% of oxidative dye precursors by total weight of the hair coloring composition.

The hair coloring composition may be substantially free of oxidizing agent.

### Direct dye

The hair coloring composition may further comprise one or more direct dyes, advantageously one or more oxidatively stable direct dyes.

The hair coloring composition may comprise a total amount from 0.001% to 4%, or from 0.005% to 3%, or from 0.01% to 2% of the one or more direct dyes by total weight of the hair coloring composition.

The presence of one or more direct dyes and the proportion thereof can help to provide or enhance coloring/dyeing, particularly with regard to the vibrancy of the color that is desired.

The hair coloring composition may be substantially free of any direct dyes. Indeed, sometimes consumers prefer direct dye-free compositions.

The one or more direct dyes may be selected from the group consisting of nitro dyes to provide a blue color, nitro dyes to provide either a red color or a yellow color, quinone dyes, basic dyes, neutral azo dyes, acid dyes, and mixtures thereof. The one or more direct dyes may be a basic dye. The one or more direct dyes may be a neutral azo dye. The one or more direct dyes may be an acid dye.

The one or more direct dyes may be selected from the group consisting of Acid dyes such as Acid Yellow 1, Acid Orange 3, Acid Black 1, Acid Black 52, Acid Orange 7, Acid Red 33, Acid Yellow 23, Acid Blue 9, Acid Violet 43, Acid Blue 16, Acid Blue 62, Acid Blue 25, Acid Red 4, Basic Dyes such as Basic Brown 17, Basic Red 118, Basic Orange 69, Basic Red 76, Basic Brown 16, Basic Yellow 57, Basic Violet 14, Basic Blue 7, Basic Blue 26, Basic Red 2, Basic Blue 99, Basic Yellow 29, Basic Red 51, Basic Orange 31, Basic Yellow 87, Basic Blue 124, 4-(3-(4-amino-9,10-dioxo-9,10-dihydroanthracen-1-ylamino)propyl)-4-methylmorpholin-4-ium-methylsulfate, (E)-1-(2-(4-(4,5-dimethylthiazol-2-yl)diazenyl)phenyl)(ethyl)amino)ethyl)-3-methyl-1H-imidazol-3-ium chloride, (E)-4-(2-(4-(dimethylamino)phenyl)diazenyl)-1-methyl-1H-imidazol-3-ium-3-yl)butane-1-sulfonate, (E)-4-(4-(2-methyl-2-phenylhydrazono)methyl)pyridinium-1-yl)butane-1-sulfonate, N,N-dimethyl-3-(4-(methylamino)-9,10-dioxo-4a,9,9a, 10-tetrahydroanthracen-1-ylamino)-N-propylpropan-1-aminium bromide, Disperse Dyes such as Disperse Red 17, Disperse Violet 1, Disperse Red 15, Disperse Black 9, Disperse Blue 3, Disperse Blue 23, Disperse Blue 377, Nitro Dyes such as 1-(2-(4-nitrophenylamino)ethyl)urea, 2-(4-methyl-2-nitrophenylamino)ethanol, 4-nitrobenzene-1,2-diamine, 2-nitrobenzene-1,4-diamine, Picramic acid, HC Red No. 13, 2,2'-(2-nitro-1,4-phenylene)bis(azanediyl)diethanol, HC Yellow No. 5, HC Red No. 7, HC Blue No.2, HC Yellow No. 4, HC Yellow No. 2, HC Orange No. 1, HC Red No. 1, 2-(4-amino-2-chloro-5-nitrophenylamino)ethanol, HC Red No. 3, 4-amino-3-nitrophenol, 4-(2-hydroxyethylamino)-3-nitrophenol, 2-amino-3-nitrophenol, 2-(3-(methylamino)-4-nitrophenoxy)ethanol, 3-(3-amino-4-nitrophenyl)propane-1,2-diol, HC Yellow No. 11, HC Violet No. 1, HC Orange No. 2, HC Orange No. 3, HC Yellow No. 9, HC Red No. 10, HC Red No. 11, 2-(2-hydroxyethylamino)-4,6-dinitrophenol, HC Blue No. 12, HC Yellow No. 6, HC Yellow No. 12, HC Blue No. 10, HC Yellow No. 7, HC Yellow No. 10, HC Blue No. 9, 2-chloro-6-(ethylamino)-4-nitrophenol, 6-nitropyridine-2,5-diamine, HC Violet No. 2, 2-amino-6-chloro-4-nitrophenol, 4-(3-hydroxypropylamino)-3-nitrophenol, HC Yellow No. 13, 6-nitro-1,2,3,4-tetrahydroquinoxaline, HC Red No. 14, HC Yellow No. 15, HC Yellow No. 14, N2-methyl-6-nitropyridine-2,5-diamine, N1-allyl-2-nitrobenzene-1,4-diamine, HC Red No. 8, HC Green No.1, HC Blue No. 14, and Natural dyes such as Annato, Anthocyanin, Beetroot, Carotene, Capsanthin, Lycopene, Chlorophyll, Henna, Indigo, Cochineal, and mixtures thereof.

### Other ingredients

The hair coloring composition according to the present invention may comprise, in addition to the ingredients indicated above, further ingredients in order to further enhance the properties of the hair coloring composition, as long as these are not excluded by the claims.

Suitable further ingredients may include, but not limited to: pigments, coloured material, solvents, radical scavengers, peroxymonocarbonate ions, surfactants, thickening agents, conditioning agents (such as silicones and cationic polymers), cosmetically acceptable carrier, preservatives, perfume and mixtures thereof.

Suitable further ingredients referred to above, but not specifically described below, are listed in the International Cosmetics Ingredient Dictionary and Handbook, (8th ed.; The Cosmetics, Toiletry, and Fragrance Association). Particularly, vol. 2, sections 3 (Chemical Classes) and 4 (Functions), which are useful in identifying specific adjuvants to achieve a particular purpose or multipurpose. A few of these ingredients are discussed hereinbelow, whose disclosure is of course non-exhaustive.

### Pigment

The hair coloring composition may comprise one or more pigments. The one or more pigments of the hair coloring composition may be a colored pigment which imparts color effects to the hair coloring composition or to the hair.

Alternatively, the one or more pigments of the hair coloring composition may be a lustre effect pigment which imparts desirable and aesthetically pleasing lustre effects to the hair coloring composition or to the keratin fibers of the hair. The color or lustre effects on the keratin fibers of the hair are preferably temporary. Indeed, the color or lustre effects on the keratin fibers of the hair last until the next hair wash and can be removed again by washing the hair with customary shampoos.

The hair coloring composition may be substantially free of pigment. Indeed, having a hair coloring composition substantially free of pigment can help to prevent the formation of residues, precipitation and/or rough hair feel.

The hair coloring composition may comprise one or more pigments having a D₅₀ particle diameter of from 5 µm to 60 µm measured according to the following test method. Particle diameter is represented by D₅₀, which is the median diameter by volume. D₅₀ is measured with a Malvern Mastersizer 2000, which is a laser diffraction particle sizer and it is measured according to ISO 13320:2009(en) with Hydro 2000G or Hydro 2000S, wherein the dispersant is water or ethanol. Detection range is from 0.02 µm to 2000 µm. D₅₀ is expressed as x₅₀ in ISO 13320:2009(en). Laser diffraction measures particle size distributions by measuring an angular variation in intensity of light scattered as a laser beam passes through a dispersed particulate sample analyser and the particle size is reported as a volume equivalent sphere diameter. A discussion of calculating D₅₀ is provided in Barber et al, Pharmaceutical Development and Technology, 3(2), 153-161 (1998), which is incorporated herein by reference.

The hair coloring composition may comprise a pigment having a D₅₀ particle diameter from 10 µm to 40 µm. The one or more pigments of the hair coloring composition may be present in the hair coloring composition in an undissolved form. The hair coloring composition may comprise from 0.01% to 25%, or from 0.1% to 20%, or from 1% to 15%, or from 4% to 10% of the one or more pigments by total weight of the hair coloring composition.

The one or more pigments of the hair coloring composition may be a colorant which is virtually insoluble in the hair coloring composition, and may be inorganic or organic. Inorganic-organic mixed pigments may be also possible. The hair coloring composition may comprise an inorganic pigment. The advantage of an inorganic pigment is its excellent resistance to light, weather and temperature. The inorganic pigment of the hair coloring composition may be of natural origin, and may be, for example, derived from a material selected from the group consisting of chalk, ochre, umber, green earth, burnt sienna, and graphite.

The one or more pigments of the hair coloring composition may be a white pigment, such as, for example, titanium dioxide or zinc oxide. Alternatively, the one or more pigments of the hair coloring composition may be a black pigment, such as, for example, iron oxide black. Alternatively, the one or more pigments of the hair coloring composition may be a colored pigment, such as, for example, ultra-marine or iron oxide red, or a lustre pigment, or a metal effect pigment, or a pearlescent pigment, and/or a fluorescent or phosphorescent pigment.

The one or more pigments of the hair coloring composition may be colored or a non-white pigment. The one or more pigments of the hair coloring composition may be selected from the group consisting of metal oxides, hydroxides and oxide hydrates, mixed phase pigments, sulfur-containing silicates, metal sulfides, complex metal cyanides, metal sulfates, chromates and molybdates, the metals themselves (bronze pigments), and combinations thereof. The one or more pigments of the hair coloring composition may be selected from the group consisting of are titanium dioxide (CI 77891), black iron oxide (CI 77499), yellow iron oxide (CI 77492), red and brown iron oxide (CI 77491), manganese violet (CI 77742), ultramarine (sodium aluminium sulfosilicates, CI 77007, Pigment Blue 29), chromium oxide hydrate (CI 77289), Prussian blue (ferric ferrocyanide, CI 77510), carmine (cochineal), and combinations thereof.

The one or more pigments of the hair coloring composition may be a pearlescent and colored pigment based on mica which is coated with a metal oxide or a metal oxychloride, such as titanium dioxide or bismuth oxychloride, and optionally further color-imparting substances, such as iron oxides, Prussian blue, ultramarine, and carmine. The color exhibited by the pigment may be adjusted by varying the layer thickness. Such pigments are sold, for example, under the trade names Rona^{®}, Colorona^{®}, Dichrona^{®}, RonaFlair^{®}, Ronastar^{®}, Xirona^{®} and Timiron^{®} all of which are available from Merck, Darmstadt, Germany. For example, Xirona^{®} is a brand for color travel pigments that display color shifting effects depending on the viewing angle and are based on either natural mica, silica or calcium aluminium borosilicate flakes, coated with varying layers of titanium dioxide.

Pigments from the line KTZ^{®} from Kobo Products, Inc., 3474 So. Clinton Ave., So. Plainfield, USA, may be also useful herein, in particular the Surface Treated KTZ^{®} Pearlescent Pigments from Kobo. Particularly useful are KTZ^{®} FINE WHITE (mica and TiO₂ having a D₅₀ particle diameter from 5 µm to 25 µm and also KTZ^{®} CELESTIAL LUSTER (mica and TiO₂, from 10 µm to 60 µm) as well as KTZ^{®} CLASSIC WHITE (mica and TiO₂, from 10 µm to 60 µm). Another useful pigment may be SynCrystal Sapphire from Eckart Effect Pigments, which is a blue powder comprising platelets of synthetic fluorphlogopite coated with titanium dioxide, ferric ferrocyanide and small amounts of tin oxide. Another useful pigment may also be SYNCRYSTAL Almond also from Eckart, which is a beige powder with a copper reflection color and is composed of platelets of synthetic fluorphlogopite and coated with titanium dioxide and iron oxides. Another useful pigment may be Duocrome^{®} RV 524C from BASF, which provides a two color look via a lustrous red powder with a violet reflection powder due to its composition of mica, titanium dioxide and carmine.

The one or more pigments of the hair coloring composition may be an organic pigment. The organic pigment of the hair coloring composition may be selected from the group consisting of natural pigments sepia, gamboge, bone charcoal, Cassel brown, indigo, chlorophyll and other plant pigments.

The one or more pigments of the hair coloring composition may be a synthetic organic pigment. The synthetic organic pigment of the hair coloring composition may be selected from the group consisting of azo pigments, anthraquinoids, indigoids, dioxazine, quinacridone, phthalocyanine, isoindolinone, perylene and perinone, metal complex, alkali blue, diketopyrrolopyrrole pigments, and combinations thereof.

The one or more pigments of the hair coloring composition may be selected from the group consisting of iron oxide, titanium dioxide, mica, borosilicate, and combinations thereof. The pigment of the hair coloring composition may comprise an iron oxide (Fe₂O₃) pigment. The one or more pigments of the hair coloring composition may comprise a combination of mica and titanium dioxide.

### Colored material

The hair coloring composition may comprise one or more colored materials. The one or more colored materials of the hair coloring composition may be particulate in form. The one or more colored materials of the hair coloring composition may be selected from the group consisting of colored fibers, colored beads, colored particles such as nano-particles, colored polymers comprising covalently attached dyes, liquid crystals, particles having diffraction properties, UV absorber and photoprotective substances, pressure- or light-sensitive pigments, and combinations thereof.

The hair coloring composition may be substantially free of colored material. Indeed, having a hair coloring composition substantially free of colored material can help to prevent the formation residues and precipitation.

The one or more colored materials of the hair coloring composition may be capable of changing color via a mechanism selected from the group consisting of thermochromism, photochromism, hydrochromism, magnetochromism, electrochromism, piezochromism, chemichromism, mechano-optics. Suitable colored material of the hair coloring composition may include 3D Magnetic Pigments, Glow Dust, Fluorescent Pigments, Thermo Dust, Chameleon Pigments and other color changing materials from Solar Color Dust (http://solarcolordust.com/).

The hair coloring composition may comprise one or more photoprotective substances. The hair coloring composition may comprise from 0.01% to 10%, or from 0.1% to 5%, or from 0.2% to 2% of the one or more photoprotective substances by total weight of the hair coloring composition. Useful photoprotective substances of the hair coloring composition are specified in European Patent Application 1084696A1 from §0036 to §0053, which is incorporated herein by reference. The one or more photoprotective substances of the hair coloring composition may be selected from the group consisting of 2-ethylhexyl 4-methoxycinnamate, methyl methoxycinnamate, 2-hydroxy-4-methoxybenzophenone-5-sulfonic acid, polyethoxylated p-aminobenzoates, di-butyl-hydroxytoluene (BHT), and mixtures thereof.

The hair coloring composition may comprise from 0.01% to 10%, or from 0.05% to 5% of one or more particulate substances by total weight of the hair coloring composition. The one or more particulate substances of the hair coloring composition may be a substance which is solid at room temperature (23°C) and in the form of a particle. The one or more particulate substances of the hair coloring composition may be selected from the group consisting of silica, silicates, aluminates, clay earths, mica, and insoluble salts. The one or more particulate substances of the hair coloring composition may be selected from the group consisting of insoluble inorganic metal salts, metal oxides, minerals and insoluble polymer particles. The one or more particulate substances of the hair coloring composition may be titanium dioxide.

The one or more particulate substances of the hair coloring composition may be present in the hair coloring composition in an undissolved, or a stably dispersed form, and, following application to the hair and evaporation of the solvent, can deposit on the hair in a solid form.

The one or more particulate substances of the hair coloring composition may be selected from the group consisting of silica (silica gel, silicon dioxide) and metal salts, in particular inorganic metal salts. The particulate substance of the hair coloring composition may be silica. The one or more particulate substances of the hair coloring composition may be selected from the group consisting of metal salts such as alkali metal or alkaline earth metal halides, e.g. sodium chloride or potassium chloride; alkali metal or alkaline earth metal sulfates, such as sodium sulfate or magnesium sulfate.

### Solvent

The hair coloring composition according to the present invention may further comprise one or more solvents. The one or more solvents may be selected from water, or a mixture of water and at least one organic solvent to dissolve the compounds that would not typically be sufficiently soluble in water.

Suitable organic solvents for the hair coloring composition may include, but are not limited to: from C₂ to C₄ lower alkanols (such as ethanol, propanol, isopropanol); aromatic alcohols (such as benzyl alcohol and phenoxyethanol); polyols and polyol ethers (such as carbitols, 2-butoxyethanol, propylene glycol, propylene glycol monomethyl ether, diethylene glycol monoethyl ether, monomethyl ether, hexylene glycol, glycerol, ethoxy glycol, butoxydiglycol, ethoxydiglycerol, dipropyleneglocol, polygylcerol); propylene carbonate; and mixtures thereof.

The one or more solvents of the hair coloring composition may be selected from the group consisting of water, ethanol, propanol, isopropanol, glycerol, 1,2-propylene glycol, hexylene glycol, ethoxy diglycol, and mixtures thereof.

Typically, the hair coloring composition may comprise water as a main ingredient, particularly in a total amount ranging from at least 50%, alternatively from at least 60%, alternatively from at least 70%, by total weight of the hair coloring composition. Typically, when present, the hair coloring composition may comprise a total amount of organic solvents ranging from 1% to 30%, by total weight of the hair coloring composition.

### Radical scavenger

The hair coloring composition may comprise one or more radical scavengers. The one or more radical scavengers of the hair coloring composition may be present in a sufficient amount to reduce damage to the hair during an oxidative bleaching or coloring process.

The one or more radical scavengers may be a species that can react with a radical species, preferably a carbonate radical to convert the radical species by a series of fast reactions to a less reactive species. The one or more radical scavengers may be advantageously selected such that the one or more radical scavengers are different from an alkalising agent and/or is present in an amount sufficient to reduce the damage to the hair during the coloring /bleaching process.

The one or more radical scavengers may be selected from the classes of: alkanolamines, amino sugars, amino acids, esters of amino acids, and mixtures thereof. Alternatively, the one or more radical scavengers may be selected from the group consisting of: monoethanolamine, 3-amino-1-propanol, 4-amino-1-butanol, 5-amino-1-pentanol, 1-amino-2-propanol, 1-amino-2-butanol, 1-amino-2-pentanol, 1-amino-3-pentanol, 1-amino-4-pentanol, 3-amino-2-methylpropan-1-ol, 1-amino-2-methylpropan-2-ol, 3-aminopropane-1,2-diol, glucosamine, N-acetylglucosamine, glycine, arginine, lysine, proline, glutamine, histidine, sarcosine, serine, glutamic acid, tryptophan, and potassium, sodium and ammonium salts of the above, and mixtures thereof. The one or more radical scavengers of the hair coloring composition may be selected from the group consisting of: benzylamine, glutamic acid, imidazole, di-tert-butylhydroxytoluene, hydroquinone, catechol, and mixtures thereof.

### Peroxymonocarbonate ions

The hair coloring composition according to the present invention may further comprise a source of carbonate ions, carbamate ions, hydrogen carbonate ions, and mixtures thereof in a sufficient amount to reduce damage to the hair during the coloring process.

The hair coloring composition may preferably comprise at least one source of peroxymonocarbonate ions. Peroxymonocarbonate ions may be formed in situ from a source of hydrogen peroxide and a carbonate ion source. The hair coloring composition may comprise a source of carbonate ions or carbamate ions or hydrocarbonate ions or any mixtures thereof. The source of peroxymonocarbonate ions may be selected from the group consisting of sodium, potassium, guanidine, arginine, lithium, calcium, magnesium, barium, ammonium salts of carbonate, carbamate and hydrocarbonate ions, and mixtures thereof.

The carbonate ion source for peroxymonocarbonate ions may be selected from the group consisting of sodium carbonate, sodium hydrogen carbonate, potassium carbonate, potassium hydrogen carbonate, guanidine carbonate, guanidine hydrogen carbonate, lithium carbonate, calcium carbonate, magnesium carbonate, barium carbonate, ammonium carbonate, ammonium hydrogen carbonate, and mixtures thereof. Percarbonate salts may also be utilized to provide both the source of carbonate ions and oxidizing agent. The source of carbonate ions, carbamate and hydrocarbonate ions may be selected from the group consisting of: sodium hydrogen carbonate, potassium hydrogen carbonate, ammonium carbamate, and mixtures thereof.

### Surfactant

The hair coloring composition may comprise one or more surfactants. A surfactant can help to provide an emulsion. The hair coloring composition may be in the form of an emulsion.

The hair coloring composition may be in the form of a cream or gel. The hair coloring composition may have a lamellar structure and/or may have a gel network. The hair coloring composition may comprise micelles comprising a hydrophobic phase (see the description of the hydrophobic phase more below).

The hair coloring composition may comprise from 0.001% to 10%, or from 0.1% to 8%, or from 0.5% to 5%, or from 0.4% to 2%, or from 0.8% to 1.5% of the one or more surfactants by total weight of the hair coloring composition.

The hair coloring composition may comprise one or more surfactants which are selected from the group consisting of anionic surfactants, non-ionic surfactants, amphoteric surfactants, zwitterionic surfactants, cationic surfactants, and mixtures thereof. The one or more surfactants of the hair coloring composition can be useful for stabilising a hydrophobic phase in the hair coloring composition, e.g. for stabilising the gel network and/or lamellar structure.

The hair coloring composition may comprise an anionic surfactant. The anionic surfactant of the hair coloring composition may be sodium lauryl sulfate or sodium laureth sulfate.

The one or more surfactants of the hair coloring composition may be a non-ionic surfactant. The non-ionic surfactant may be selected from the group consisting of lanolin alcohol, and polyoxyethylene ethers of fatty alcohols, and mixtures thereof. The non-ionic surfactant may be preferably ceteareth-n, wherein n is from 2 to 100, or from 10 to 30. When the one or more surfactants of the hair coloring composition are non-ionic, precipitation of others ingredients of the hair coloring composition can be prevented.

The hair coloring composition may comprise from 0.001% to 5%, or from 0.01% to 3%, or from 0.01% to 1%, or from 0.05% to 1%, or from 0.1% to 0.5%, or from 0.1% to 0.3% of a non-ionic surfactant by total weight of the hair coloring composition. The non-ionic surfactant of the hair coloring composition may be selected from the group consisting of lanolin alcohol, and polyoxyethylene ethers of fatty alcohols, and mixtures thereof.

The non-ionic surfactant of the hair coloring composition may be a castor oil having polyethylene glycol ether groups or polypropylene glycol ether groups. The polyethylene glycol ether groups of the non-ionic surfactant may be ethers of PEG-n groups, wherein n is an integer of from 2 to 12, or from 2 to 10, or from 3 to 8. When the total M.Wt. of polyethylene glycol ether groups is below 400 Da, the mixing of the hair coloring composition can be eased.

The polypropylene glycol ether groups may be ethers of PPG-n groups, wherein n is an integer of from 2 to 60, or from 10 to 50, or from 20 to 40. The polyethylene glycol ether groups or polypropylene glycol ether groups may be selected from the group consisting of: PPG-4, PPG-6, PEG-5, PEG-6, PEG-8, and mixtures thereof. The hair coloring composition may comprise PEG-40 Hydrogenated Castor Oil and/or PEG-60 Castor Oil and/or PEG-35 Castor Oil as non-ionic surfactant.

### Thickening agent

The hair coloring composition may comprise one or more thickening agents. Thickening agents can help to provide the desired rheology for the hair coloring composition, which is useful in terms of mixing and anti-drip. The hair coloring composition may comprise from 0.01% to 5% of the one or more thickening agents by total weight of the hair coloring composition. The one or more thickening agents of the hair coloring composition may be a thickening polymer.

The hair coloring composition may comprise from 0.1% to 2% of a thickening polymer by total weight of the hair coloring composition. The thickening polymer of the hair coloring composition may be selected from the group consisting of associative polymers, crosslinked acrylic acid homopolymers, crosslinked copolymers of (meth)acrylic acid and of (C₁-C₆) alkyl acrylate, polysaccharides and mixtures thereof. The thickening polymer of the hair coloring composition may also serve as conditioning agents, as described below.

### Conditioning agent

The hair coloring composition may comprise one or more conditioning agents. The one or more conditioning agents of the hair coloring composition may be selected from the group consisting of silicone materials, amino silicones, fatty alcohols, polymeric resins, polyol carboxylic acid esters, cationic polymers, cationic surfactants, insoluble oils and oil derived materials and mixtures thereof. The one or more conditioning agents of the hair coloring composition may be selected from the group consisting of mineral oils, glycerine, sorbitol and mixtures thereof.

The hair coloring composition may comprise from 0.05% to 20%, or from 0.1% to 15%, or from 0.2% to 10%, or from 0.2% to 2%, or from 0.5% to 2% of the one or more conditioning agents by total weight of the hair coloring composition. The one or more conditioning agents may be included in a separate pre- and/or post-treatment composition.

Suitable conditioning agents may include, but are not limited to: silicones, aminosilicones, fatty alcohols, polymeric resins, polyol carboxylic acid esters, cationic polymers, cationic surfactants, insoluble oils and oil derived materials and mixtures thereof. Additional conditioning agents may include mineral oils and other oils such as glycerin and sorbitol.

Particularly useful conditioning agents for the hair coloring composition may be cationic polymers and/or silicones. Cationic polymers may be chosen from those comprising units of at least one amine group chosen from primary, secondary, tertiary and quaternary amine groups that may either form part of the main polymer chain, or be borne by a side substituent that is directly attached to the main polymer chain.

The one or more conditioning agents of the hair coloring composition may be a silicone. The silicone of the hair coloring composition may be selected from the group consisting of polyalkylsilioxane oils, linear polydimethylsiloxane oils containing trimethylsilyl or hydroxydimethylsiloxane endgroups, polymethylphenylsiloxane polydimethylphenylsiloxane, polydimethyldiphenylsiloxane oils, silicone resins, organofunctional siloxanes having in their general structure one or a number of organofunctional group(s), the same or different, attached directly to the siloxane chain, and mixtures thereof. Said organofunctional group(s) may be selected from: polyethyleneoxy and/or polypropyleneoxy groups, (per)fluorinated groups, thiol groups, substituted or unsubstituted amino groups, carboxylate groups, hydroxylated groups, alkoxylated groups, quaternium ammonium groups, amphoteric, betain groups and mixtures thereof. The silicone of the hair coloring composition may be either used as a neat fluid or in the form of an pre-formed emulsion.

### Cosmetically acceptable carrier

The hair coloring composition comprises a cosmetically acceptable carrier. The cosmetically acceptable carrier of the hair coloring composition may be an aqueous carrier. The hair coloring composition may comprise water. Water can provide a hydrophilic phase, which the hydrophilic portions of any other ingredients comprised in the hair coloring composition can interact with water. Water can also provide a fluid phase meaning that the hair coloring composition can be in liquid form and therefore easily mixed with other fluid formulations such as the oxidizing formulation. The hair coloring composition may comprise from 50% to 85% water, or from 65% to 75% of water by total weight of the hair coloring composition.

The cosmetically acceptable carrier is any carrier suitable for formulating the one or more acrylic compounds into a hair coloring composition being suitable for application onto hair. The cosmetically acceptable carrier may be selected from either an aqueous medium or an aqueous-alcoholic medium. When the cosmetically acceptable carrier is an aqueous-alcoholic carrier, the cosmetically acceptable carrier may comprise water and an alcohol. An alcohol can advantageously influence the viscosity of a relatively wide spectrum of ingredients of the hair coloring composition. The alcohol of the hair coloring composition may be selected from the group consisting of ethanol, isopropanol, propanol, and mixtures thereof.

When the cosmetically acceptable carrier is an aqueous carrier, the aqueous carrier may consist essentially of water and may be substantially free of alcohol. The hair coloring composition may comprise a safe and effective amount of a cosmetically acceptable carrier which is water. The hair coloring composition may comprise from 0.1% to 99%, or from 1% to 98%, or from 10% to 97%, or from 30% to 95% of water by total weight of the hair coloring composition.

The hair coloring composition may be substantially free of alcohol, such as volatile alcohols (e.g. ethanol, isopropanol, propanol). When the hair coloring composition is substantially free of alcohol, the hair coloring composition can advantageously have a reduced odour. Flammability issues can also be prevented.

The cosmetically acceptable carrier of the hair coloring composition may be an oily compound. The oily compound may be selected from the group consisting of cyclic silicones and volatile hydrocarbons. Cyclic silicones can be available from Dow Corning. The cyclic silicone may have from at least 3 silicone atoms or from at least 5 silicone atoms but no more than 7 silicone atoms or no more than 6 silicone atoms. The cyclic silicone may conform to the formula: 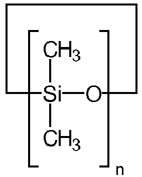 wherein n is from 3 or from 5 but no more than 7 or no more than 6. The cyclic silicone may have a kinematic viscosity of less than 10 cSt at 23°C. A Suitable cyclic silicone for use herein may include Cyclomethicone D5 (commercially available from G.E. Silicones). Alternatively, the hair coloring composition may be silicone-free.

Volatile hydrocarbons e.g. Isopar can be obtained from ExxonMobil Petroleum and Chemical. The oily compiound may be a mineral oil. Trade names for suitable mineral oils include Benol, Blandol, Hydrobrite, Kaydol (Sonneborn LLC Refined Products), Chevron Superla White Oil (Chevron Products Company), Drakeol, Parol (Calumet Penreco LLC), Peneteck (Calumet Penreco LLC), Marcol, and Primol 352 (ExxonMobil Petroleum and Chemical).

### Hydrophobic phase

The hair coloring composition may comprise a hydrophobic phase. The hydrophobic phase of the hair coloring composition may be selected from the group consisting of fatty alcohols, fatty acids, and mixtures thereof. The fatty alcohols and/or fatty acids may comprise from 10 to 30, or from 12 to 20, or from 16 to 18 carbon atoms. The hydrophobic phase of the hair coloring composition may comprise two different fatty alcohols. The hydrophobic phase of the hair coloring composition may comprise two different fatty alcohols, both comprising from 10 to 14 carbons.

### Preservative

The hair coloring composition may comprise at least one preservative and/or a mixture of preservatives. The hair coloring composition may comprise from 0.01% to 1% preservative, or from 0.1% to 0.5% preservative by total weight of the hair coloring composition. The preservative of the hair coloring composition may be selected from the group consisting of benzyl alcohol, phenoxyethanol, 1,3-bis(hydroxymethyl)-5,5-dimethylimidazolidine-2,4-dione, and mixtures thereof. The hair coloring composition may comprise at least one preservative; and wherein the preservative may be selected from the group consisting of benzyl alcohol, phenoxyethanol, and mixtures thereof; or wherein the preservative may be a mixture of benzyl alcohol and phenoxyethanol. The hair coloring composition may be substantially free of benzoate compounds. Indeed, having benzoate compounds can help to prevent instability and/or precipitation of the hair coloring composition. The hair coloring composition may be substantially free of parabens.

### Perfume

The hair coloring composition may comprise a perfume. The hair coloring composition may comprise from 0.001% to 2% of a perfume by total weight of the hair coloring composition. Perfume can provide an enhanced user experience by making the composition smell pleasant and/or invoke emotions tailored to the visual effects on the hair fibers, such as relaxing or exciting smells.

Alternatively, the hair coloring composition may be substantially free of perfume and/or fragrance. Some consumers prefer perfume-free compositions.

The perfume of the hair coloring composition may be an animal fragrance or a plant fragrance. The animal fragrance may be selected from the group consisting of musk oil, civet, castoreum, ambergris, and mixtures thereof.

The plant fragrance may be selected from the group consisting of nutmeg extract, cardomon extract, ginger extract, cinnamon extract, patchouli oil, geranium oil, orange oil, mandarin oil, orange flower extract, cedarwood, vetyver, lavandin, ylang extract, tuberose extract, sandalwood oil, bergamot oil, rosemary oil, spearmint oil, peppermint oil, lemon oil, lavender oil, citronella oil, chamomille oil, clove oil, sage oil, neroli oil, labdanum oil, eucalyptus oil, verbena oil, mimosa extract, narcissus extract, carrot seed extract, jasmine extract, olibanum extract, rose extract, and mixtures thereof.

The perfume of the hair coloring composition may be selected from the group consisting of acetophenone, adoxal, aldehyde C-12, aldehyde C-14, aldehyde C-18, allyl caprylate, ambroxan, amyl acetate, dimethylindane derivatives, α-amylcinnamic aldehyde, anethole, anisaldehyde, benzaldehyde, benzyl acetate, benzyl alcohol and ester derivatives, benzyl propionate, benzyl salicylate, borneol, butyl acetate, camphor, carbitol, cinnamaldehyde, cinnamyl acetate, cinnamyl alcohol, cis-3-hexanol and ester derivatives, cis-3-hexenyl methyl carbonate, citral, citronnellol and ester derivatives, cumin aldehyde, cyclamen aldehyde, cyclo galbanate, damascones, decalactone, decanol, estragole, dihydromyrcenol, dimethyl benzyl carbinol, 6,8-dimethyl-2-nonanol, dimethyl benzyl carbinyl butyrate, ethyl acetate, ethyl isobutyrate, ethyl butyrate, ethyl propionate, ethyl caprylate, ethyl cinnamate, ethyl hexanoate, ethyl valerate, ethyl vanillin, eugenol, exaltolide, fenchone, fruity esters such as ethyl 2-methyl butyrate, galaxolide, geraniol and ester derivatives, helional, 2-heptonone, hexenol, α-hexylcinnamic aldehyde, hydroxycitrolnellal, indole, isoamyl acetate, isoeugenol acetate, ionones, isoeugenol, isoamyl iso-valerate, iso E super, limonene, linalool, lilial, linalyl acetate, lyral, majantol, mayol, melonal, menthol, p-methylacetophenone, methyl anthranilate, methyl cedrylone, methyl dihydrojasmonate, methyl eugenol, methyl ionone, methyl-α-naphthyl ketone, methylphenylcarbinyl acetate, mugetanol, γ-nonalactone, octanal, phenyl ethyl acetate, phenylacetaldehyde dimethyl acetate, phenoxyethyl isobutyrate, phenyl ethyl alcohol, pinenes, sandalore, santalol, stemone, thymol, terpenes, triplal, triethyl citrate, 3,3,5-trimethylcyclohexanol, γ-undecalactone, undecenal, vanillin, veloutone, verdox, and mixtures thereof.

### Viscosity

The hair coloring composition may have a kinematic viscosity of from 0.5 cSt to 1500 cSt, measured at 23°C according to the following method. "Viscosity" can mean dynamic viscosity (measured in mPa·s) or kinematic viscosity (measured in centistokes, cSt) of a liquid at 23°C and ambient conditions. Dynamic viscosity may be measured using a rotational viscometer, such as a Brookfield Dial Reading Viscometer Model 1-2 RVT available from Brookfield Engineering Laboratories (USA) or other substitutable model as known in the art. Typical Brookfield spindles which may be used include, without limitation, RV-7 at a spindle speed of 20 rpm, recognizing that the exact spindle may be selected as needed by one skilled in the art. Kinematic viscosity may be determined by dividing dynamic viscosity by the density of the liquid (at 23°C and ambient conditions), as known in the art.

The viscosity of the hair coloring composition may be useful in view of enabling the hair coloring composition to be readily applied to the hair fibers - e.g. spread evenly onto the hair. Viscosity can be influenced by the level of cosmetically acceptable carrier in the hair coloring composition and the level of the thickening agent.

The hair coloring composition may have a kinematic viscosity of from 1 cSt to 1000 cSt. The hair coloring composition may have a kinematic viscosity of from 1.5 cSt to 500 cSt, or from 2 cSt to 350 cSt, or from 2.5 cSt to 200 cSt, or from 3 cSt to 150 cSt, measured at 23°C. 1 centistoke (cSt) is equal to 1 x 10⁻⁶ m²/s).

The hair coloring composition may have a dynamic viscosity of from 1 mPa·s to 5000 mPa·s. The hair coloring composition may have a viscosity of from 2 mPa·s to 400 mPa·s, or from 3 mPa·s to 100 mPa·s. Alternatively, the hair coloring composition may have a dynamic viscosity of from 30 mPa·s to 250 mPa·s, or from 100 mPa·s to 200 mPa·s.

This viscosity range of the hair coloring composition may be useful in order to prevent the hair coloring composition from dripping. When the viscosity is relatively too high, the hair coloring composition may not be readily mixed, e.g. with the cosmetically acceptable carrier.

### Volatility

The hair coloring composition may be substantially free of compounds having a vapor pressure below 0.01 mmHg, or below 0.001 mm Hg, measured at 23°C and 1 atm. Having a hair coloring composition having a relatively low volatility can help to reduce the odour of the hair coloring composition and also can help to provide a relatively safer safety profile.

### Use

A second aspect of the present invention relates to the use of the one or more acrylic compounds of Formula I in a hair coloring composition as stated hereinbefore. The one or more acrylic compounds of Formula I have a molecular weight of less than 500 g/mol, preferably less than 300 g/mol, more preferably less than 250 g/mol, or even more preferably less than 100 g/mol.

The use of the one or more acrylic compounds of Formula I can help to improve the color result intensity or the vibrancy of the hair color. The use of the one or more acrylic compounds of Formula I can promote a relatively more concentrated dyeing in the outer portion of the hair fibers.

### Kit

A third aspect of the present invention is related to a kit for coloring hair comprising:
(a) a hair coloring composition according to the first aspect;
(b) an oxidizing formulation comprising one or more oxidizing agents; wherein the hair coloring composition (a) and the oxidizing formulation (b) are separately packaged.

The kit may further comprise (c) a conditioning formulation comprising one or more conditioning agents. Conditioning agents have already been described above.

The kit may further comprise (d) a thickening formulation. Such thickening formulations are currently on the market as under the brand "Color.id" from Wella™ Professionals. The thickening formulation of the kit may comprise one or more thickening polymers capable of interacting with the hydrophobic phase and the hydrophilic phase.

The thickening formulation of the kit may comprise from 0.001% to 10.0%, or from 0.01%, or 0.05%, or 0.1%, or 0.15%, or 0.25%, or 0.6%, or 1%, or 2%, or 2.5% to 8%, or 7%, or 6%, or 5%, or 4%, or 3%, or 2%, or 1% of the one or more thickening polymers by total weight of the thickening formulation. The one or more thickening polymers of the thickening formulation may be an associative thickening polymer.

The kit may further comprise a mixing receptacle and/or a mixing means. The mixing receptacle of the kit may be a bowl. The mixing means of the kit may be a spatula.

The oxidizing formulation of the kit may comprise one or more oxidizing agents. Preferred oxidizing agents are water-soluble peroxygen oxidizing agents. The one or more oxidizing agents can be valuable for the initial solubilisation and decolorisation of the melanin (bleaching) and accelerate the oxidation of the oxidative dye precursors (oxidative dyeing) in the hair shaft.

The one or more oxidizing agents may be present in an amount sufficient to bleach melanin pigment in hair and/or cause formation of dye chromophores from oxidative dye precursors. Typically, the oxidizing formulation of the kit may comprise a total amount of oxidizing agents ranging from 0.1% to 20%, or from 0.5% to 12%, or from 1% to 10%, or from 2% to 5%, by total weight of the oxidizing formulation of the kit.

Suitable water-soluble oxidizing agents may include, but are not limited to: inorganic peroxygen materials capable of yielding hydrogen peroxide in an aqueous solution.

Suitable water-soluble peroxygen oxidizing agents may include, but are not limited to: hydrogen peroxide; inorganic alkali metal peroxides (such as sodium periodate and sodium peroxide); organic peroxides (such as urea peroxide and melamine peroxide); inorganic perhydrate salt bleaching compounds (such as the alkali metal salts of perborates, percarbonates, perphosphates, persilicates, persulfates and the like); and mixtures thereof. Inorganic perhydrate salts may be incorporated for example as monohydrates, tetrahydrates. Alkyl/aryl peroxides and/or peroxidases may also be used. Mixtures of two or more such oxidizing agents can be used if desired. The oxidizing agents may be provided in aqueous solution or as a powder which is dissolved prior to use.

The oxidizing formulation of the kit may preferably comprise a water-soluble oxidizing agent which is selected from the group consisting of hydrogen peroxide, percarbonates (which may be used to provide a source of both oxidizing agent and carbonate ions), persulfates, and mixtures thereof. The one or more oxidizing agents of the oxidizing formulation of the kit may be sodium percarbonate. The oxidizing formulation of the kit may be substantially free of persulfate.

The hair coloring composition (a) and the oxidizing formulation (b) may be packaged in separate sealed containers. The hair coloring composition (a) may be packaged in a flexible tube packaging composed of metal, plastics or a combination thereof. The oxidizing formulation (b) may be packaged in a squeezable container. The squeezable container may have at least 50% headspace. The squeezable container may have a headspace being at least the volume of the hair coloring composition (a). The oxidizing formulation may be packaged in a plastic container according to claim 1 of European Patent Application EP 2 801 281 A1, wherein the plastic container has two symmetrical collapsible side panels and a non-collapsible squeezable back panel; wherein the ratio of the average thicknesses between front and/or back panels and the side panels is at least 2:1 (EP 2 801 281 A1 paragraphs [0025] to [0044] as well as the Figures are incorporated herein by reference). The plastic container has the advantage that it is resistant to random, uncontrolled deformation under a substantial pressure differential between the environment and inside the container, yet having an affordable cost of manufacture and/or being appealing to the consumer.

### Method

A fourth aspect of the present invention is related to a method of coloring hair comprising:
(a) mixing the hair coloring composition according to the first aspect with an oxidizing formulation comprising one or more oxidizing agents to form a mixture; and
(b) applying the mixture onto hair.
Steps (a) and (b) are carried out in the order (a) and then (b).

The mixture in the method may be obtained from mixing the hair coloring composition according to the first aspect and the oxidizing formulation at a 1:1 ratio by weight.

### Rheology

The hair coloring formulation may further comprise a hydrophobic phase, a hydrophilic phase, one or more surfactants, and one or more thickening polymers capable of interacting with the hydrophobic phase and the hydrophilic phase, wherein the composition has a storage modulus of at least 3000 Pa, or at least 3300 Pa, or at least 3500 Pa, or at least 4000 Pa, or at least 4500 Pa, or at least 5000 Pa, measured by frequency sweep at an angular frequency of 0.6 rad/s at 23°C, and wherein the one or more thickening polymers are an associative thickening polymer and comprise hydrophobic moieties and hydrophilic moieties. The storage modulus may be not more than 10 kPa, or 9 kPa, or 8 kPa, or 7 kPa, or 6kPa, measured by frequency sweep at an angular frequency of 0.6 rad/s at 23°C. The hydrophilic moieties of the associative thickening polymer may comprise urethane units.

### EXAMPLES

The following examples are non-limiting examples of the hair coloring compositions of the present invention. The examples are given solely for the purpose of illustration, and are not to be construed as limitations of the present invention since many variations thereof are possible without departing from the spirit and scope of the invention, which would be recognized by one of ordinary skill in the art. All concentrations are listed as weight percent (% wt.), unless otherwise specified.

### Hair coloring composition examples 1 to 7

The following hair coloring compositions are prepared.

| **Ingredients** | **Ex.1** (% wt.) | **Ex**.**2** (% wt.) | **Ex**.**3** (% wt.) | **Ex**.**4** (% wt.) | **Ex.5** (% wt.) | **Ex**.**6** (% wt.) | **Ex.7** (% wt.) |
|---|---|---|---|---|---|---|---|
| 2-propenic acid * | 11.0 | 10.0 | - | - | 9.0 | 5.0 | 12.0 |
| 2-methylpropenic acid + | - | - | 9.0 | 12.0 | - | 6.0 | - |
| Cetyl alcohol | 7.0 | 14.0 | - | 7.0 | - | 6.0 | 6.5 |
| Stearyl alcohol | 7.0 | - | 12.0 | 7.0 | - | 6.0 | 6.5 |
| Sodium laureth-6 carboxylate | - | - | - | - | - | 2.0 | - |
| Coconut alcohol | - | - | 0.2 | - | - | 0.5 | - |
| Propylene glycol | | - | - | - | 25.0 | - | 10.0 |
| Sodium myreth sulfate | - | - | - | - | - | 2.0 | - |
| Glyceryl stearate | 4.0 | 3.5 | 5.0 | 4.0 | - | - | - |
| Glyceryl oleate | 4.0 | 3.5 | - | 4.0 | | - | - |
| PEG-7 Glyceryl Cocoate | 5.0 | 5.5 | - | 5.0 | - | - | 2.0 |
| 2,5-diaminotoluene sulfate | 2.1 | 1.8 | - | 2.1 | - | 1.5 | 1.5 |
| 2,4-diamino phenoxyethanol HCl | - | 0.01 | 2.0 | - | - | - | - |
| 1-methyl-2,5-diamino benzene | - | - | - | - | 1.7 | - | - |
| N,N-*bis*(2-hydroxyethyl)-p-phenylenediamine sulfate | - | - | 2.5 | - | - | - | - |
| 2-amino-4-hydroxyethylamino anisole sulfate monohydrate | - | - | 1.5 | - | - | - | - |
| 2-methyl-5-hydroxyethyl aminophenol | - | - | 1.0 | - | - | - | - |
| 3-amino-2,6-dimethylphenol | - | - | 2.0 | | - | - | - |
| 2-amino-5-ethylphenol HCl | - | - | 1.0 | - | - | - | - |
| Hydroxyethyl-3,4-methylenedioxyaniline HCl | - | - | 0.5 | - | - | - | - |
| 1,4-diamino-2-(methoxymethyl)-benzene | - | - | 1.8 | - | 4.0 | - | - |
| Phenyl methyl pyrazolone | - | - | 0.25 | | - | - | - |
| 1-hydroxyethyl-4,5-diamino pyrazole sulfate | - | - | 3.0 | - | - | 3.0 | 3.0 |
| 4-amino-2-hydroxytoluene | 0.1 | - | 0.5 | 0.1 | 1.4 | 1.0 | 1.0 |
| m-aminophenol | 0.1 | 0.2 | 0.5 | 0.1 | - | 0.1 | - |
| p-aminophenol | - | - | 0.5 | - | 2.5 | - | 0.5 |
| Resorcinol | 1.2 | 0.8 | 1.3 | 1.2 | 0.9 | - | - |
| 2-amino-6-chloro-4-nitrophenol | - | - | 1.0 | - | - | - | - |
| 5-amino-6-chloro-o-cresol | - | - | - | - | - | - | 1.0 |
| 2-methylresorcinol | - | - | 1.0 | - | - | - | - |
| 1-naphthol | - | - | 1.0 | - | - | - | - |
| Lauryl glucoside | - | - | - | - | - | 2.0 | - |
| Decyl glucoside | | | - | - | - | - | |
| Acrylamido propyltrimonium chloride/acrylates copolymer | - | - | - | - | - | 1.0 | - |
| Carbomer | | | - | - | - | - | 0.5 |
| Hydroxyethylcellulose | - | - | - | - | 1.5 | - | - |
| Polyquaternium-22 | - | - | 0.05 | - | - | - | 0.1 |
| Polyquaternium-10 | 0.3 | 0.3 | - | 0.3 | - | - | - |
| Polyquaternium-7 | 3.0 | 3.0 | - | 3.0 | - | - | - |
| Dimethicone | - | - | - | - | - | - | 0.5 |
| Ceteareth-12 | - | - | - | - | - | 0.5 | - |
| Ceteareth-20 | - | 1.0 | - | - | - | 0.5 | - |
| Ceteareth-25 | 1.0 | - | - | 1.0 | - | - | - |
| Laureth-12 | - | - | - | - | - | - | 1.0 |
| Deceth-3 | - | - | - | - | - | - | 1.0 |
| Oleth-30 | - | - | - | - | - | - | 1.0 |
| Sodium Laureth Sulfate | 2.0 | 2.0 | 1.0 | 2.0 | | - | - |
| Sodium Lauryl Sulfate | 2.0 | - | 2.0 | 2.0 | - | - | - |
| Macadamia ternifolia seed oil | - | - | - | - | - | 0.1 | - |
| Grape seed oil | - | - | - | - | - | - | 0.1 |
| Sodium sulfite | 0.4 | - | 0.2 | 0.4 | - | 0.2 | - |
| Sodium metabisulfite | - | 0.3 | - | - | 0.7 | - | 0.5 |
| Ammonium sulfate | - | 0.1 | - | - | - | 0.1 | - |
| Sodium sulfate | 0.5 | - | 0.5 | 0.5 | - | - | - |
| Pentasodium pentetate | - | - | - | - | - | - | 0.1 |
| Silica dimethyl silylate (nano) | - | - | - | - | - | - | 0.1 |
| Sodium silicate | - | - | - | - | - | 0.1 | - |
| Etidronic acid | 0.2 | 0.2 | - | 0.2 | - | 0.2 | - |
| Disodium EDTA | - | - | 0.05 | - | 0.1 | - | - |
| Titanium dioxide | - | - | - | - | - | - | 0.1 |
| Monoethanol amine | - | - | - | - | 14.5 | - | 16 |
| Ascorbic acid | 0.2 | 0.2 | 0.15 | 0.2 | 0.3 | 0.2 | 0.2 |
| Lauric acid | - | - | - | - | - | 0.15 | 0.1 |
| Sodium chloride | - | - | - | - | - | 0.1 | 0.1 |
| Potassium hydroxide | - | - | - | - | - | 0.2 | - |
| Sodium hydroxide | 0.03 | - | 0.25 | 0.03 | | - | - |
| Ammonium hydroxide | 2.0 | 2.3 | 1.25 | 2.0 | - | 2.0 | 1.5 |
| Perfume | 0.25 | 0.2 | 0.2 | 0.25 | - | 0.2 | 0.1 |
| Water | QSP | QSP | QSP | QSP | QSP | QSP | QSP |
| Total | 100 | 100 | 100 | 100 | 100 | 100 | 100 |

| | | | | | | | |
|---|---|---|---|---|---|---|---|
| KEY: *= acrylic acid; ⁺ = methacrylic acid. | | | | | | | |

The hair coloring composition is mixed with a developer composition at a mixing of 1:1. A suitable developer composition is a commercially available Wella Welloxon™ Perfect Developer Cream, 6% wt. hydrogen peroxide. The resulting mixtures are then applied to hair swatches of hair (3 g per hair swatches). After a leave-on time of 30 min at room temperature (23°C), the hair is rinsed, washed with a standard shampoo, rinsed and then dried.

### EXPERIMENTAL

### Color variation test method

The colorimetric parameters in the CIE L* a* b* system were measured using an Ocean Optics USB 2000+ spectrophotometer. (illuminant D65, angle 10°, specular component included) in which L* represents the lightness of the color, a* indicates the green/red color axis and b* the blue/yellow color axis. The L*, a* and b* values were average values, the measurement being performed on about 80 different positions of two hair swatches by the spectrophotometer and averaged.

### Tint compositions (oxidative dye precursors)

### Three tint compositions T1-T3 were first prepared.

Composition T1 was a 10 g hair coloring composition comprising a mixture of oxidative dye precursors prepared from a composition comprising 100% wt. (i.e. 10 g) of a commercially available Wella Koleston™ Perfect 6/0 (dark blond color) by total weight of the hair coloring composition.

Composition T2 was a 10 g hair coloring composition comprising a mixture of oxidative dye precursors prepared from a composition comprising 85% wt. (i.e. 8.5 g) of a commercially available Wella Koleston^{TM} Perfect 6/0 (dark blond color) by total weight of the hair coloring composition and 15% wt. (i.e. 1.5 g) of water.

Composition T3 was a 10 g hair coloring composition comprising a mixture of oxidative dye precursors prepared from a composition comprising 70% wt. (i.e. 7.0 g) of a commercially available Wella Koleston^{TM} Perfect 6/0 (dark blond color) by total weight of the hair coloring composition and 30% wt. (i.e. 3.0 g) of water.

### Hair coloring compositions

Six hair coloring compositions A-F were prepared:
Composition A was prepared by mixing 10 g of composition T 1 with 10 g of water.
Composition B was prepared by mixing 10 g of composition T2 with 10 g of water.
Composition C was prepared by mixing 10 g of composition T3 with 10 g of water.
Composition D was prepared by mixing 10 g of composition T1 with 10 g of a 4% wt. aqueous solution of acrylic acid.
Composition E was prepared by mixing 10 g of composition T2 with 10 g of a 4% wt. aqueous solution of acrylic acid.
Composition F was prepared by mixing 10 g of composition T3 with 10 g of a 4% wt. aqueous solution of acrylic acid.
The 4% wt. aqueous solution of acrylic acid has been purchased from Sigma-Aldrich.

### Hair swatches

Hair tresses having a width of 2.5 cm and a length of 22 cm.
Available from *International Hair Importers* & *Products*, Glendale, NY
Mas: 3.0 g ± 0.05g
Characteristics: cysteic acid: 17.4- 18.1 µmol/g hair; medullated hair, φ: 60-80 µm

### Pre-treatment (Bleaching process)

The hair swatches were weighed and then soaked in a mixture of 2.5 g bleaching powder and 7.5 ml Welloxyd™ per 1 g of hair (in a lab basin). It has to be carried out in an extractor hood. The concentration of hydrogen peroxide in Welloxyd™ was 9%. The residence time during the bleaching step was 30 min. The hair swatches were turned upside down after each 7.5 min. After bleaching, the hair swatches were rinsed under tap water (6 L/min, 35°C) for 2 min. The bleaching process was carried out twice in which the second bleaching step followed immediately after the first bleaching step. Before the 2^{nd} bleaching step was started, the hair was dabbed with a napkin. Subsequently, the hair swatches were washed twice with 0.25 ml standard-shampoo (10% Na-laurylether-sulfate, 4% NaCl) per 1 g hair for 1 minute, rinsed for 1 minute. Then, the hair swatches were stored in distilled water for 24h. After that the hair swatches were rinsed for 2 min under tap water (6 L/min, 35°C). Finally, the hair swatches were dried at 20°C and 65% relative humidity at least overnight.

### Coloring treatment

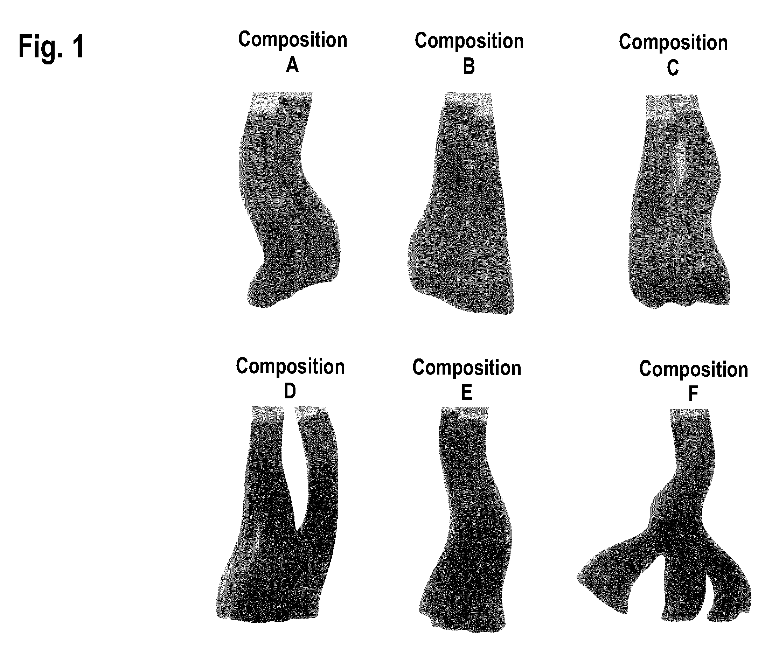

Each composition A-F was mixed with 10 g of a developer composition which was the commercially available Wella Welloxon™ Perfect Developer Cream, 6% wt. hydrogen peroxide. Then, 3 g of each composition A-F to be tested was applied with a brush to two pre-treated hair swatches. The color for each hair swatch was left for 30 minutes at 30°C. After 30 minutes, each hair swatch was rinsed for 2 min under tap water (6 L/min, 35°C) and then blow-dried. The resulted hair swatches treated with each composition A-F provided an overall dark blond color (Fig. 1).

After 30 minutes the hair coloring composition is rinsed off the hair for 2 minutes and the hair is then shampooed with Wella™ Professionals Brillance, Fine/Normal shampoo commercially available in Germany in April 2014 and conditioned with Wella™ Professionals Brillance, Fine/Normal conditioner commercially available in Germany in April 2014 for 1 minute and then blow-dried.

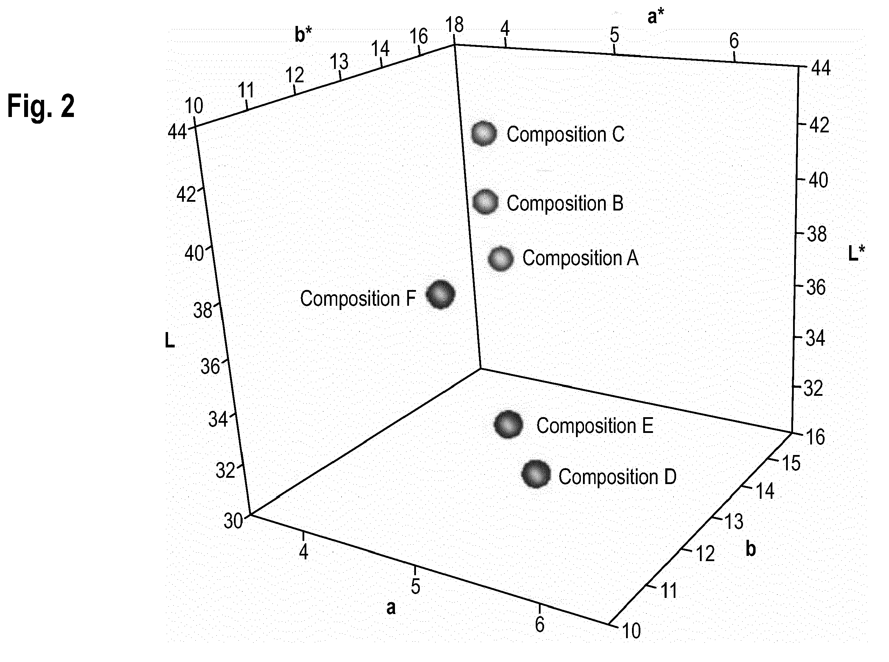

The colorimetric parameters in the CIE L* a* b* system for each hair swatch were measured according to the Color variation test method. The resulting color for each hair swatch were characterized by the CIE L* a* b* system. In particular, a L* value of 100 is considered white and a L* value of 0 is considered black, therefore the higher the L* value the lower the color intensity. The L* a* b* values were measured for each compositions A-F on two hair swatches as shown in Fig. 1 and averaged. The measured L* a* b* values for each composition A-F were reported on the CIE L* a* b* color space, as shown in Fig. 2.

### Results

Fig. 1 shows pairs of hair swatches that have been pre-treated with a bleaching process and then treated with the respective compositions A-F. The Compositions A, B and C do not comprise any acrylic acid and thus are control compositions. The compositions D, E and F comprise acrylic acid as the acrylic compound of the hair coloring composition of the present invention.

When 10 g water is replaced with 10 g of a 4% wt. aqueous solution of acrylic acid, the addition of acrylic acid darkens the hair swatches. In Fig. 1, the hair swatches treated with compositions D, E and F are darker than the hair swatches treated with the respective controlled compositions A, B and C.

Fig. 2 shows the CIE L* a* b* color space obtained by measuring the L* a* b* values for two hair swatches that have been treated with each respective composition A-F as shown in Fig. 1. The a* and b* values measured on hair swatches treated either with the controlled composition or the respective composition comprising acrylic acid are within the same range. Hence, it can be derived that no new color species have been formed. In other words, the resulting "dark blond color" is formed with the same chromophore species.

However, the L* values measured on hair swatches treated with the controlled composition in comparison with the respective composition comprising acrylic acid vary significantly. For instance, the L* value of hair swatch treated with Composition D (comprising composition T1 and acrylic acid) is much lower than the L* value of hair swatch treated with controlled Composition A (comprising composition T1 and water).

Also, the hair swatches treated with Composition F (comprising composition T3, 75% wt. (i.e. 7.5 g) of a commercially available Wella Koleston™ Perfect 6/0) can have a similar color than the hair swatches treated with Composition A (comprising composition T1, 100% wt. (i.e. 10 g) of a commercially available Wella Koleston™ Perfect 6/0) Indeed, in each case, the measured a* b* values are in the same range.

### Conclusion

The presence of acrylic acid in the compositions D, E and F can provide a decrease of the L* value of the resulting color, which provides a darker color and an increased of color intensity. Hence, the presence of acrylic acid in a hair coloring composition can help to darken the resulting color and to enhance the color vibrancy.

Since the Composition F (comprising composition T3, 75% wt. (i.e. 7.5 g) of a commercially available Wella Koleston^{TM} Perfect 6/0 with acrylic acid) can provide a similar color than the Composition A (comprising composition T1, 100% wt. (i.e. 10 g) of a commercially available Wella Koleston^{TM} Perfect 6/0), the presence of acrylic acid in a hair coloring composition can help to decrease the proportion of oxidative dye precursors in the hair coloring composition, which can lead to a substantial cost saving.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A hair coloring composition comprising, in a cosmetically acceptable carrier:
one or more acrylic compounds of Formula I, or a cosmetically acceptable salt thereof, or a mixture thereof: 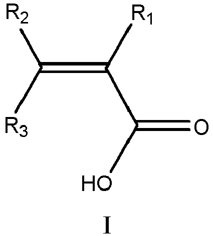 wherein
R₁ is selected from the group consisting of -H, -CH₃, -CF₃, -C₂H₅, -C₃H₈, -iPr, -C₄H₉, -sBu, -iBu, tBu-, and -CH₂-COOCH₃;
R₂ is selected from the group consisting of -H, -CH₃, -C₂H₅, -C₃H₈, -iPr, -C₄H₉, -sBu, -iBu, tBu-, -OCH₃, -COOH, and -CH₂COOH;
R₃ is selected from the group consisting of -H, and -CH₃;
wherein the one or more acrylic compounds of Formula I have a molecular weight of less than 500 g/mol, preferably less than 300 g/mol, more preferably less than 250 g/mol or even more preferably less than 100 g/mol;
wherein the hair coloring composition comprises oxidative dye precursors comprising one or more couplers and one or more primary intermediates; and
wherein the hair coloring composition has a pH of from 8 to 12.

2. The hair coloring composition of Claim 1, wherein the hair coloring composition comprises from 0.5% to 50%, preferably from 0.5% to 15% of the one or more acrylic compounds of Formula I, or a cosmetically acceptable salt thereof, or a mixture thereof by total weight of the hair coloring composition.

3. The hair coloring composition according to any of the preceding claims, wherein
R₁ is selected from the group consisting of -H, -CH₃, -CF₃, -C₂H₅, -C₃H₈, -iPr, -C₄H₉, -sBu, -iBu, tBu-, and -CH₂-COOCH₃;
R₂ is H;
R₃ is H.

4. The hair coloring composition of Claim 3,
wherein
R₁ is selected from the group consisting of -H, and -CH₃;
R₂ is H;
R₃ is H.

5. The hair coloring composition according to any of the preceding claims wherein the cosmetically acceptable salt is selected from the group consisting of sodium salt, potassium salt, ammonium salt, calcium salt, magnesium salt, iron salt, zinc salt, zirconium salt, aluminium salt and mixtures thereof.

6. The hair coloring composition according to any of the preceding claims, wherein the one or more primary intermediates are selected from the group consisting of toluene-2,5-diamine, p-phenylenediamine, N-phenyl-p-phenylenediamine, N,N-bis(2-hydroxyethyl)-p-phenylenediamine, 2-hydroxyethyl-p-phenylenediamine, hydroxypropyl-bis-(N-hydroxyethyl-p-phenylenediamine), 2-methoxymethyl-p-phenylenediamine, 2-(1,2-dihydroxyethyl)-p-phenylenediamine, 2,2'-(2-(4-aminophenylamino)ethylazanediyl)diethanol, 2-(2,5-diamino-4-methoxyphenyl)propane-1,3-diol, 2-(7-amino-2H-benzo[b][1,4]oxazin-4(3H)-yl)ethanol, 2-chloro-p-phenylenediamine, p-aminophenol, p-(methylamino)phenol, 4-amino-m-cresol, 6-amino-m-cresol, 5-ethyl-o-aminophenol, 2-methoxy-p-phenylenediamine, 2,2'-methylenebis-4-aminophenol, 2,4,5,6-tetraminopyrimidine, 2,5,6-triamino-4-pyrimidinol, 1-hydroxyethyl-4,5-diaminopyrazole sulfate, 4,5-diamino-1-methylpyrazole, 4,5-diamino-1-ethylpyrazole, 4,5-diamino-1-isopropylpyrazole, 4,5-diamino-1-butylpyrazole, 4,5-diamino-1-pentylpyrazole, 4,5-diamino-1-benzylpyrazole, 2,3-diamino-6,7-dihydropyrazolo[1,2-a]pyrazol-1(5H)-one dimethosulfonate, 4,5-diamino-1-hexylpyrazole, 4,5-diamino-1-heptylpyrazole, methoxymethyl-1,4-diaminobenzene, N,N-bis(2-hydroxyethyl)-N-(4-aminophenyl)-1,2-diaminothane, 2-[(3-aminopyrazolo[1,5-a]pyridin-2-yl)oxy]ethanol hydrochloride, salts thereof and mixtures thereof.

7. The hair coloring composition according to any of the preceding claims, wherein the one or more primary intermediates are 1,4-diamino-2-(methoxymethyl)-benzene.

8. The hair coloring composition according to any of the preceding claims, wherein the hair coloring composition is substantially free of oxidizing agent.

9. The hair coloring composition according to any of the preceding claims, wherein the hair coloring composition comprises a total amount from 0.001% to 12% of oxidative dye precursors by total weight of the hair coloring composition.

10. The hair coloring composition according to any of the preceding claims, wherein the hair coloring composition further comprises one or more direct dyes, preferably one or more oxidatively stable direct dyes.

11. The hair coloring composition according to claim 10, wherein, the hair coloring composition comprises a total amount from 0.001% to 4% of the one or more direct dyes by total weight of the hair coloring composition.

12. The hair coloring composition according to any of the preceding claims, wherein the hair coloring composition is substantially free of any initiator of free-radical polymerisation.

13. The hair coloring composition according to any of the preceding claims, wherein the hair coloring composition is substantially free of polyacrylic polymer derivative obtained from the one or more acrylic compounds.

14. Use of one or more acrylic compounds of Formula I or a cosmetically acceptable salt thereof, or a mixture thereof: 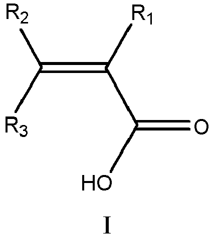 wherein
R₁ is selected from the group consisting of -H, -CH₃, -CF₃, -C₂H₅, -C₃H₈, -iPr, -C₄H₉, -sBu, -iBu, tBu-, and -CH₂-COOCH₃;
R₂ is selected from the group consisting of -H, -CH₃, -C₂H₅, -C₃H₈, -iPr, -C₄H₉, -sBu, -iBu, tBu-, -OCH₃, -COOH, and -CH₂COOH;
R₃ is selected from the group consisting of -H, and -CH₃;
in an hair coloring composition, wherein the one or more acrylic compounds of Formula I have a molecular weight of less than 500 g/mol, for improving the vibrancy of a hair color.

15. A kit for coloring hair comprising:
(a) a hair coloring composition as defined in claims 1 to 13;
(b) an oxidizing formulation comprising one or more oxidizing agents; and
wherein the hair coloring composition (a) and the oxidizing formulation (b) are separately packaged.

16. A method of coloring hair comprising:
(a) mixing a hair coloring composition as defined in claims 1 to 13 with an oxidizing formulation comprising one or more oxidizing agents to form a mixture; and
(b) applying the mixture onto hair.
